(19) Europäisches Patentamt
European Patent Office
Office européen des brevets

(11) Publication number: **0 008 021**
**B1**

(12) **EUROPEAN PATENT SPECIFICATION**

(45) Date of publication of patent specification: **16.05.84**

(21) Application number: **79102497.9**

(22) Date of filing: **17.07.79**

(51) Int. Cl.³: **C 07 D 487/14**, C 12 P 17/18 // A61K31/40, A61K35/70, C11D3/386

(54) **New mitomycins and processes for production thereof.**

(30) Priority: **18.07.78 JP 86748/78**
**13.03.79 JP 28250/79**
**28.06.79 JP 80809/79**

(43) Date of publication of application:
**20.02.80 Bulletin 80/04**

(45) Publication of the grant of the patent:
**16.05.84 Bulletin 84/20**

(84) Designated Contracting States:
**DE FR GB IT**

(56) References cited:
**US-A-3 627 781**
**US-A-3 629 283**
**US-A-3 738 998**

**The file contains technical information submitted after the application was filed and not included in this specification**

(73) Proprietor: **KYOWA HAKKO KOGYO CO., LTD**
**Ohtemachi Bldg. Ohtemachi 1-chome Chiyoda-ku**
**Tokyo (JP)**

(72) Inventor: **Kasai, Masaji**
**3-12-15, Kugenumamatsugaoka**
**Fujisawa-shi Kanagawa-ken (JP)**
Inventor: **Kono, Motomichi**
**3-6-6, Asahi-machi**
**Machida-shi Tokyo (JP)**
Inventor: **Shirahata, Kunikatsu**
**3-9-10, Naka-machi**
**Machida-shi Tokyo (JP)**
Inventor: **Urakawa, Chikahiro**
**1384, Kiso-machi**
**Machida-shi Tokyo (JP)**
Inventor: **Tsuchiya, Haruko**
**1998-96, Usuba**
**Ontahara-shi Tochigi-ken (JP)**
Inventor: **Nakano, Kinichi**
**4-23-32, Tsukushino**
**Machida-shi Tokyo (JP)**
Inventor: **Takahashi, Itaru**
**6-27, Kuwamizuhon-machi**
**Kumamoto-shi Kumamoto-ken (JP)**
Inventor: **Mineura, Kazuyuki**
**1188, Shimotogari Nagaizumi-cho**
**Sunto-gun Shizuoka-ken (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:
**JOURNAL OF MEDICINAL CHEMISTRY, Vol. 14, no. 2, 1971, Washington, S. KINOSHITA et al. "Mitomycin Derivatives. 2. Derivatives of Decarbamoylmitosane and Decarbamoylmitosene" pages 109 to 112**

**Chemical Abstracts, Volume 77, Nr. 23, December 4, 1972, (COLUMBUS, OHIO, USA), A.A. ABOU-ZEID et al. "Fermentative production of mitomycins by Streptomyces caespitosus" page 289, column 2, abstract Nr. 150 562 P**

**Chemical Abstracts, Volume 82, Nr. 21, May 26, 1975, (COLUMBUS, OHIO, USA) A.A. ABOU-ZEID et al. "Biosynthesis of mitomycins by Streptomyces caespitosus" page 444, column 2, abstract Nr. 137 684 P**

(74) Representative: **Casalonga, Alain et al**
**BUREAU D.A. CASALONGA OFFICE JOSSE & PETIT Baaderstrasse 12-14**
**D-8000 München 5 (DE)**

**Description**

This invention relates to new mitomycins having a double bond between 9-position and 10-position and processes for production thereof.

Mitomycins are generally known as compounds having anti-tumor activity. As representative mitomycins, there may be enumerated mitomycin factors A, B and C, porfiromycin (The Merck Index, 9th Ed.). Further, there are known compounds named mitomycin factors D and E on which an application has been filed by the present applicant (Japanese Patent Application No. 29238/1978). These mitomycins have the following chemical structures and are obtained by culturing a certain strain of *Streptomyces caespitosus.*

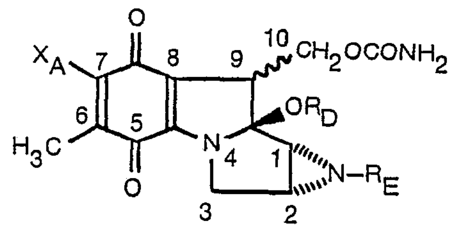

| | $X_A$ | 9 ∿∿∿ 10 | $R_D$ | $R_E$ |
|---|---|---|---|---|
| Mitomycin A | $OCH_3$ | IIIIIIII | $CH_3$ | H |
| ,, B | $OCH_3$ | ◀ | H | $CH_3$ |
| ,, C | $NH_2$ | IIIIIIII | $CH_3$ | H |
| ,, D | $NH_2$ | ◀ | H | $CH_3$ |
| ,, E | $NH_2$ | ◀ | $CH_3$ | $CH_3$ |
| Porfiromycin | $NH_2$ | IIIIIIII | $CH_3$ | $CH_3$ |

Further, various derivatives of these compounds are known. For example, 1a-N-acetyl-mitomycin C (U.S. Patent No. 3,514,452), 1a-N-butyryl-mitomycin C (U.S. Patent No. 3,514,452), 7-ethylamino-7-demethoxy-mitomycin A (U.S. Patent No. 3,514,452), 10-decarbamoyl-mitomycins A, B and C (U.S. Patent No. 3,738,998), 1a-N-acetyl-10-decarbamoyl-mitomycin C (Japanese Published Examined Patent Application No. 17,279/1974), 1a-N-acetyl-10-decarbamoyl-10-p-toluene-sulfonyl-mitomycin C (Japanese Published Examined Patent Application No. 37,639/1972), 10-decarbamoyl-10-p-toluene-sulfonyl-porfiromycin (Japanese Published Examined Patent Application No. 37,639/1972), etc. are known.

A compound having an excellent property is always in demand. The present inventors have studied mitomycins, found new mitomycins having antibacterial activities, and completed the present invention.

In accordance with the present invention, new mitomycins of the general formula [I] (hereinafter often referred to as Compound [I]. The same rule applies correspondingly to the compounds of other formulae)

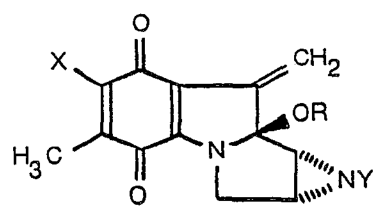

(I)

wherein X is amino, hydroxy or alkoxy having 1—4 carbon atoms; Y is hydrogen, methyl, $—COR^1$ or $—CH_2CH_2Z$

$R_1$ is hydrogen, alkyl having 1—3 carbon atoms, chloromethyl, phenyl or p-methoxyphenyl,

Z is alkoxycarbonyl having 2—3 carbon atoms, $—COR^4$ or cyano

$R^4$ is hydrogen or alkyl having 1—3 carbon atoms; R is hydrogen or alkyl having 1—4 carbon atoms and processes for production thereof are offered.

The desired compounds of the present invention (Compound [I]) have broad antibacterial activity

and are useful for purpose of sterilization of equipment, etc. in a hospital, as a washing agent with detergent or as a medicament. Further, Compound [I] is useful as an intermediate for a new mitomycin derivative.

Fig. 1 illustrates IR spectrum of 10-decarbamoyloxy-9-dehydro-mitomycin B.

Fig. 2 illustrates IR spectrum of 7-amino-10-decarbamoyloxy-9-dehydro-7-demethoxy-mitomycin B.

Fig. 3 illustrates IR spectrum of 9a-O-methyl-10-decarbamoyloxy-9-dehydro-mitomycin B.

Fig. 4 illustrates IR spectrum of 7-amino-9a-O-methyl-10-decarbamoyloxy-9-dehydro-7-demethoxy-mitomycin B.

Fig. 5 illustrates IR spectrum of 10-decarbamoyloxy-9-dehydro-mitomycin C.

Fig. 6 illustrates IR spectrum of 7-deamino-10-decarbamoyloxy-9-dehydro-7-hydroxy-mitomycin C.

Fig. 7 illustrates IR spectrum of 10-decarbamoyloxy-9-dehydro-mitomycin A.

Fig. 8 illustrates IR spectrum of 10-decarbamoyloxy-9-dehydro-7-deamino-7-hydroxy-porfiromycin.

In the group X of the general formula [I], the alkoxy group has 1—4 carbon atoms, e.g., methoxy, ethoxy, i-propoxy, n-butoxy, t-butoxy. In the group $R^1$, the alkyl group has 1—3 carbon atoms, e.g., methyl, ethyl, i-propyl.

In the group Z, the alkyl group of the term "alkoxycarbonyl" has 1—4 carbon atoms, e.g., methyl, ethyl, t-butyl, and $R^4$ is hydrogen or alkyl having 1—3 carbon atoms, e.g., methyl, ethyl, i-propyl. In the group R, the alkyl group has 1—4 carbon atoms, e.g., methyl, ethyl, n-butyl.

The following compounds are representatively exemplified as Compound [I].

| No. of compound | Name of compound | Structural formula | No. of example |
|---|---|---|---|
| 1 | 1a-N-acetyl-10-decarbamoyloxy-9-dehydromitomycin C | 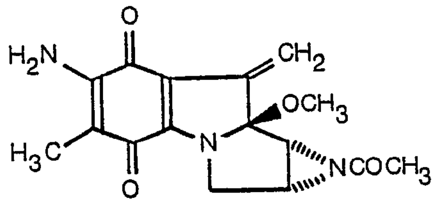 | 1 |
| 2 | 10-decarbamoyloxy-9-dehydromitomycin C | 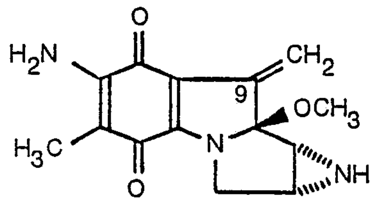 | 2, 6, 8, 18 |
| 3 | 7-deamino-10-decarbamoyloxy-9-dehydro-7-hydroxy-mitomycin C | 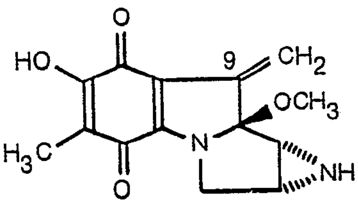 | 14 |
| 4 | 10-decarbamoyloxy-9-dehydromitomycin A | 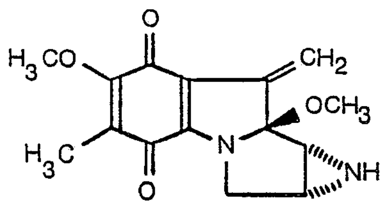 | 16 |
| 8 | 10-decarbamoyloxy-9-dehydro-1a-N-(2-formylethyl) mitomycin C | 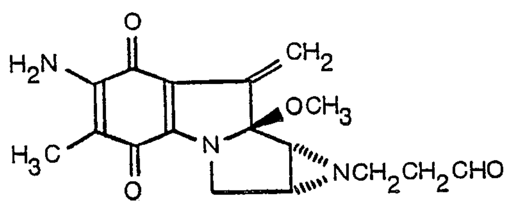 | 5 |
| 9 | 10-decarbamoyloxy-9-dehydromitomycin B | 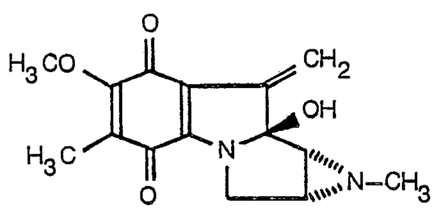 | 9, 21 |
| 10 | 9a-O-methyl-10-decarbamoyloxy-9-dehydromitomycin B | 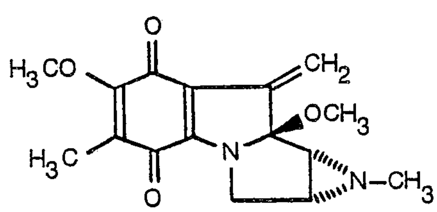 | 11, 17, 22 |

| No. of compound | Name of compound | Structural formula | No. of example |
|---|---|---|---|
| 11 | 7-amino-10-decarbamoyloxy-9-dehydro-7-demethoxymitomycin B | 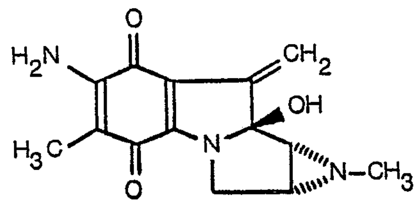 | 10 |
| 12 | 10-decarbamoyloxy-9-dehydroporfiromycin (7-amino-9a-O-methyl-10-decarbamoyloxy-9-dehydro-7-demethoxymitomycin B) | 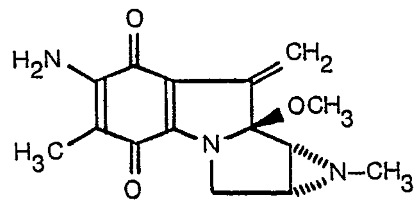 | 12, 13, 19, 20 |
| 13 | 10-decarbamoyloxy-9-dehydro-7-deamino-7-hydroxyporfiromycin | 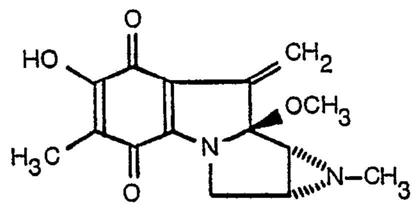 | 15 |

Physical properties of these compounds are shown in the examples.

Minimum inhibitory concentration ($\mu$g/ml) (agar dilution method, pH 7.0) against the following various bacteria is shown in Tables 1—5.

A *Serratia marcescens* ATCC 4003
B *Pseudomonas cepacia* ATCC 25608
C *Staphylococcus aureus* ATCC 6538P
D *Escherichia coli* ATCC 26
E *Bacillus subtilis* No. 10707
F *Proteus vulgalis* ATCC 6897
G *Shigella sonnei* ATCC 9290
H *Salmonella typhosa* ATCC 9992
I *Klebsiella pneumoniae* ATCC 10031

TABLE 1

| Bacteria / Test compound | A | B | C | D | E | F | G | H | I |
|---|---|---|---|---|---|---|---|---|---|
| Compound 1 | >200 | >200 | 200 | — | 6.3 | 200 | — | >200 | 50 |
| Mitomycin C | 2.5 | >10 | 0.16 | 5.0 | 0.039 | 0.078 | 1.3 | 5.0 | 0.020 |

TABLE 2

| Bacteria / Test compound | A | B | C | D | E | F | G | H | I |
|---|---|---|---|---|---|---|---|---|---|
| Compound 2 | >11 | >11 | 2.6 | – | 0.041 | 1.3 | – | >11 | 0.66 |
| Compound 3 | >8.6 | >8.6 | 4.3 | – | 0.54 | 4.3 | – | >8.6 | 4.3 |
| Compound 4 | >150 | >150 | 9.4 | >150 | 0.59 | 9.4 | – | >150 | 4.7 |
| Mitomycin C | 5.6 | 5.6 | 0.087 | >11 | 0.044 | 0.087 | 5.6 | 5.6 | 0.011 |

TABLE 3

| Bacteria / Test compound | A | B | C | D | E | F | G | H | I |
|---|---|---|---|---|---|---|---|---|---|
| Compound 8 | >50 | 50 | 13 | – | 0.39 | 25 | – | >50 | 13 |
| Mitomycin C | 2.5 | 10 | 0.039 | >10 | 0.020 | 0.039 | 5 | 2.5 | 0.0098 |

TABLE 4

| Bacteria / Test compound | C | E | G | I |
|---|---|---|---|---|
| Compound 9 | 0.782 | 0.098 | >50 | 12.5 |
| ,, 10 | 6.25 | 0.391 | >50 | 12.5 |
| ,, 11 | 3.125 | 0.098 | 25 | 3.125 |
| ,, 12 | 50 | 0.196 | >50 | 12.5 |
| Mitomycin B | <0.025 | <0.025 | 3.125 | 0.196 |
| Compound A* | 3.125 | 0.782 | 25 | 3.125 |

*7-Amino-7-demethoxy-mitomycin B (Mitomycin D).

TABLE 5

| Bacteria / Test compound | A | B | C | D | E | F | G | H | I |
|---|---|---|---|---|---|---|---|---|---|
| Compound 13 | > 200 | – | 100 | 200 | < 0.098 | 25 | 200 | 50 | 6.3 |
| " 12 | > 200 | – | 100 | – | 0.195 | 100 | > 200 | – | 3.1 |

Processes for the production of Compounds of formula [I] are exemplified below.

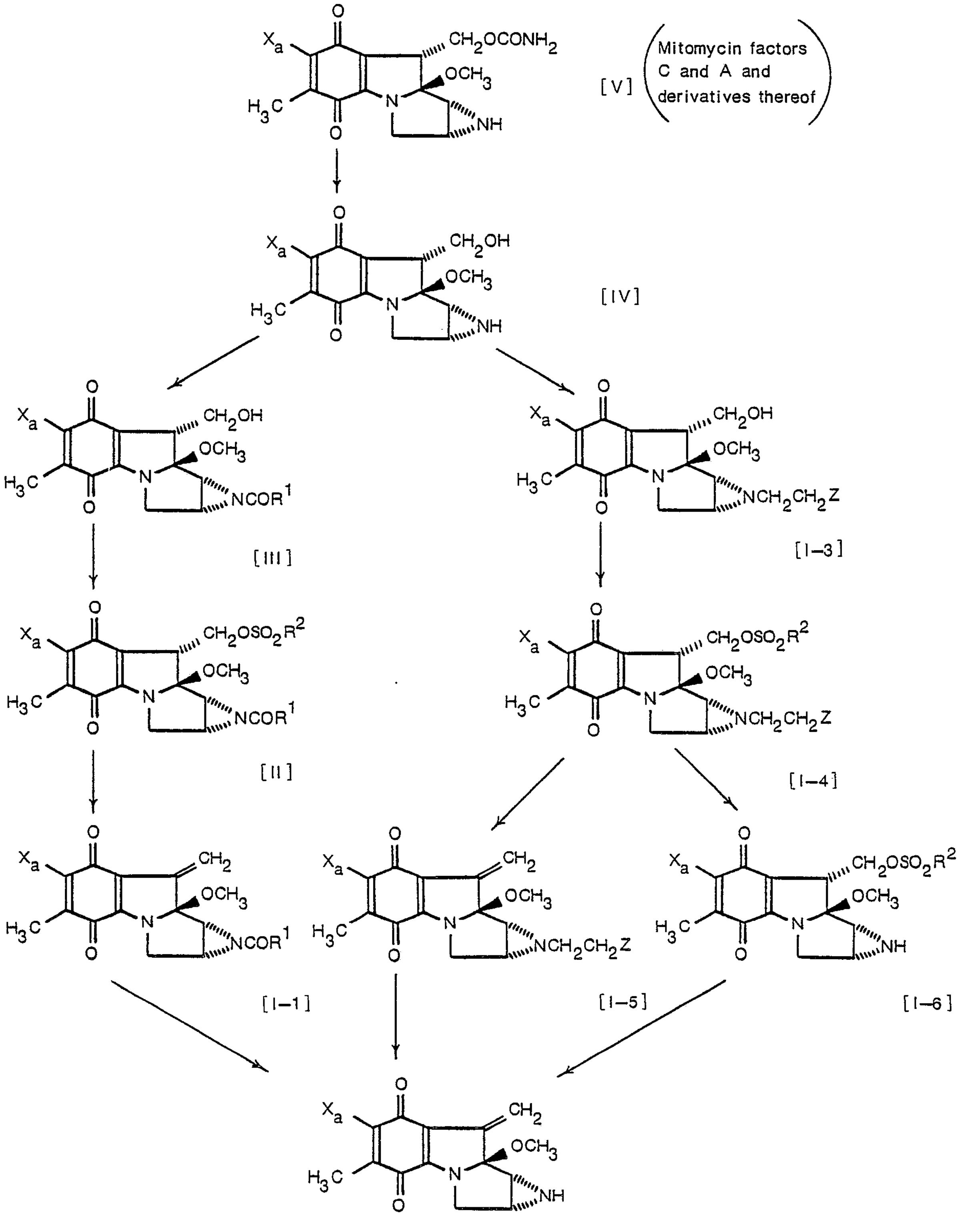


[V]

[IV]

[III]

[I–3]

[II]

[I–4]

[I–1]

[I–5]

[I–6]

[I–2]

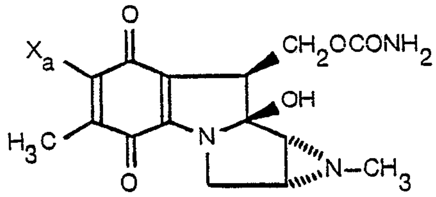

[VI] (Mitomycin factors B and D and derivatives thereof)

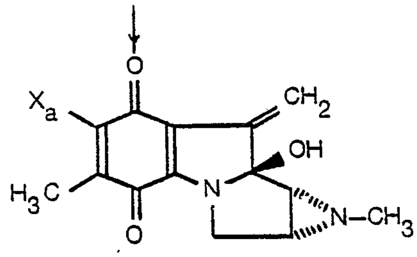

[I–7]

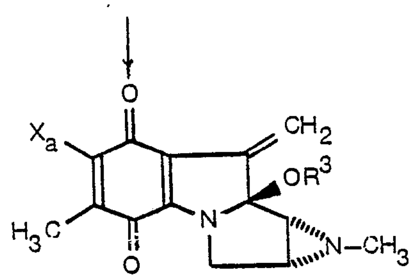

[I–8]

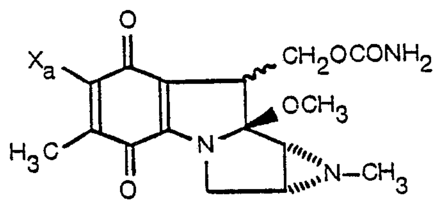

[IX] (Porfiromycin and mitomycin E and derivatives thereof)

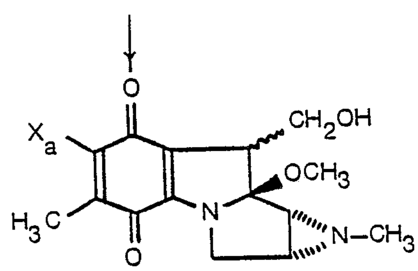

[VIII]

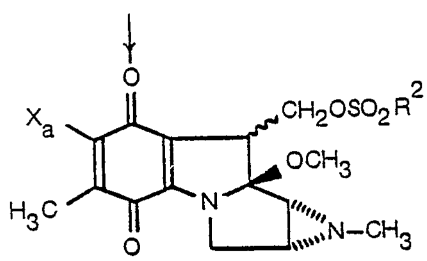

[VII]

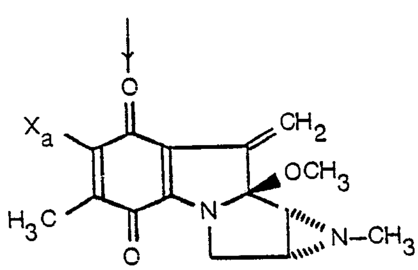

[I–8–1]

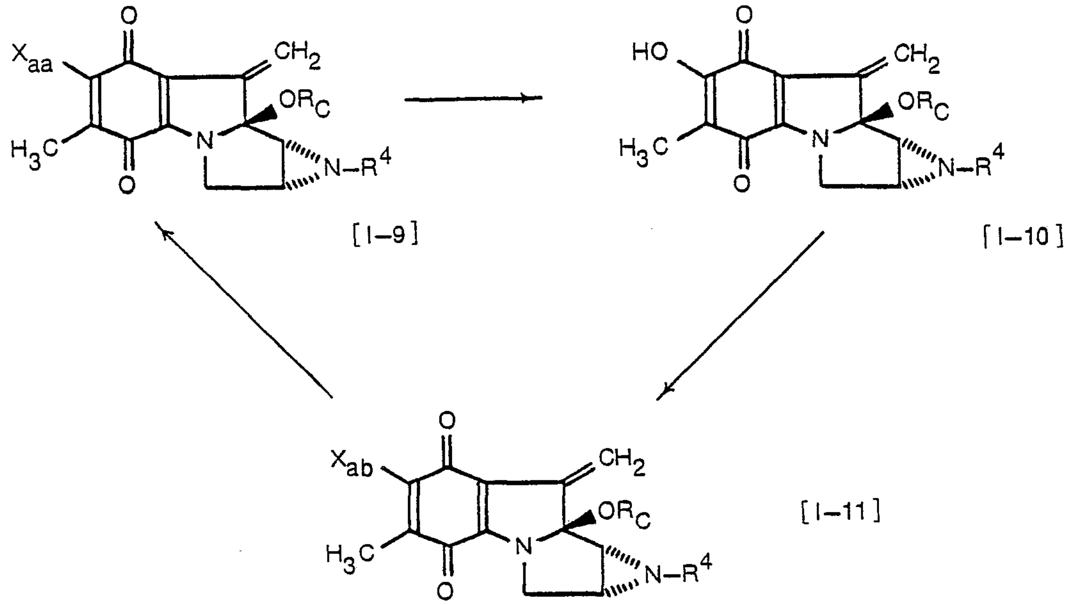

[I—9]

[I—10]

[I—11]

Fermentation 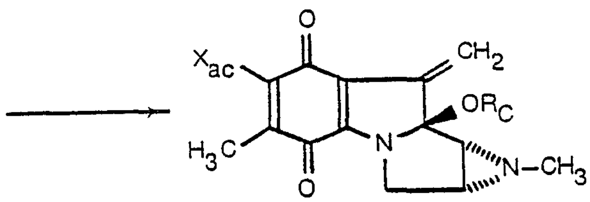 [I—12]

In the above processes, $X_a$ is amino, substituted amino or alkoxy, ($X_a$ is X wherein hydroxy is excluded). $R^3$ is alkyl ($R^3$ is $R_C$ wherein hydrogen is excluded). $X_{aa}$ is amino or substituted amino ($X_{aa}$ is $X_a$ wherein alkoxy is excluded). $X_{ab}$ is alkoxy ($X_{ab}$ is $X_a$ wherein amino and substituted amino are excluded). $R^4$ is hydrogen or methyl. $X_{ac}$ is amino or alkoxy ($X_{ac}$ is $X_a$ wherein substituted amino is excluded). $R^1$, $R^2$, Z and $R_C$ have the same meaning as defined before.

Compounds [I—1]—[I—12] are included in Compound [I]. Compound [I—8—1] is included in Compound [I—8]. Compounds [II]—[IX] are known.

Each of the above processes is described below.

### Process of Compound [V] → Compound [IV]

In Compound [V], a compound wherein $X_a$ is amino is mitomycin C and a compound wherein $X_a$ is methoxy is mitomycin A. A compound of formula [V] wherein $X_a$ is alkoxy (excluding methoxy) and a process for production thereof are disclosed in U.S. Patent No. 3,558,651. A compound of formula [V] wherein $X_a$ is substituted amino and a process for production thereof are disclosed in J. Med. Chem., *14*, 103 (1971).

Compounds of formula [IV] are also known and may be obtained by lithium aluminum hydride method (U.S. Patent No. 3,738,998), hydrolysis by alkali (U.K. Patent No. 1,250,063), sodium alcoholate method [U.K. Patent No. 1,250,063; J. Med. Chem., *64*, 109 (1971)], etc.

Sodium alcoholate method which is illustrated in Reference Example 1 is briefly described below.

Compound [IV] is obtained by eliminating carbamoyl from Compound [V] in the presence of an alcoholate in a solvent innert to the reaction.

As the alcoholate, alcoholates of methanol, ethanol, t-butanol, etc. with sodium, potassium, etc. may be used. As the solvent, methanol, ethanol, i-propanol, tetrahydrofuran, dioxane, dimethylformamide, benzene, etc. may be used.

The alcoholate is used preferably in an amount of 5 to 7 times moles of Compound [V].

The reaction is usually carried out at room temperature and completed in several hours to several days.

### Process of Compound [IV] → Compound [III]

Compound [III] is obtained by reacting Compound [IV] with a reactive derivative of a carboxylic acid represented by the general formula

$$R^1COOH$$

wherein $R^1$ has the same meaning as defined before, such as acid halide, acid anhydride, etc., (hereinafter referred to as an acylating agent), in the presence of a base, in an innert solvent.

As the acylating agent, acetyl chloride, propionyl chloride, acetic anhydride, etc. may be used. As the base, sodium carbonate, sodium hydride, triethylamine, pyridine, etc. may be used. As the solvent, tetrahydrofuran, dioxane, chloroform, etc. may be used. Certain bases, such as pyridine, may also function as the solvent.

The acylating agent is preferably used in an amount of 1 to 2 times moles of Compound [IV]. The base is preferably used in an amount of 1 to 100 times moles of Compound [IV].

The reaction is usually carried out at −78 to 30°C.

Process of Compound [III] → Compound [II]

Compound [II] is obtained by reacting Compound [III] with a reactive derivative of a compound represented by the general formula

$$R^2SO_2OH$$

wherein $R^2$ has the same meaning as defined before (hereinafter referred to as a sulfonylating agent), in the presence of a base, in an innert solvent.

As the sulfonylating agent, halides, acid anhydrides, etc. of the compound represented by the above general formula, such as methanesulfonyl chloride, trifluoromethanesulfonyl chloride, p-toluenesulfonyl chloride, etc. may be used. As the base, inorganic bases such as sodium carbonate, sodium hydride, etc. and organic bases such as triethylamine, pyridine, etc. may be used. These organic bases also function as the solvent. As the solvent, tetrahydrofuran, dioxane, chloroform, etc. may be used.

The sulfonylating agent is used in an amount of 1 to 2 times moles of Compound [III]. The base is used in an amount of 1 to 100 times moles of Compound [III].

The reaction is usually carried out at −78 to 30°C.

Process of Compound [II] → Compound [I—1]

Compound [I—1] is obtained by eliminating alkyl (or aryl)-sulfonic acid from Compound [II] in the presence of a base in an inert solvent.

As the base, inorganic bases such as sodium hydroxide, sodium hydride, etc. and organic bases such as potassium-t-butoxide, 1,5-diazabicyclo[5.4.0]undecene-5, triethylamine, sodium methoxide, especially potassium t-butoxide, 1,5-diazabicyclo[5.4.0]undecene-5, may be used. As the solvent, ethyl ether, tetrahydrofuran, dioxane, ethylene glycol dimethyl ether, n-hexane, petroleum ether, benzene, N,N-dimethylformamide, ethyl acetate, acetone, methylene chloride, dimethylsulfoxide, etc. may be used.

The base is used in an amount of 1 to 20 times moles of Compound [II]

The reaction is carried out at 0 to 80°C and is usually completed in one hour to one week.

Process of Compound [I—1] → Compound [I—2]

Compound [I—2] is obtained by hydrolyzing Compound [I—1] in the presence of a base in water and an inert solvent.

As the base, sodium hydrogen carbonate, ammonia, diethylamine, hydrazine, etc. may be used. As the innert solvent, methanol, ethanol, tetrahydrofuran, dimethylformamide, ethyl acetate, acetone, chloroform, etc. may be used.

The base is used in an amount of 1 to 20 times moles of Compound [I—1].

The reaction is carried out at 0 to 60°C and usually completed in 1 to 10 hours.

Process of Compound [IV] → Compound [I—3]

Compound [I—3] is obtained by reacting Compound [IV] with a compound represented by the general formula

$$CH_2{=}CHZ$$

wherein Z has the same meaning as defined before (hereinafter referred to as an alkylating agent) in an inert solvent to alkylate 1a-N-position of Compound [IV]

When an alkylating agent active to the reaction (for example, acrolein) is used, the reaction proceeds by only mixing the agent with Compound [IV]. However, the reaction is usually carried out in the presence of a base such as triethylamine, sodium methoxide, potassium t-butoxide, etc. for smooth proceeding of the reaction. As the solvent, ethyl ether, tetrahydrofuran, dioxane, ethylene glycol dimethyl ether, n-hexane, petroleum ether, benzene, N,N-dimethylformamide, ethyl acetate, acetone, methylene chloride, chloroform, dimethylsulfoxide, etc. may be used.

The alkylating agent is used in an amount of 1 to 30 times moles of Compound [IV].

The reaction is carried out preferably at 20 to 80°C and usually completed in one hour to one week.

Alkylating method adopted in the process is called Michael addition, which is disclosed in "Ethylenimine and Other Aziridines" 136, 1969 (Academic Press, U.S.A.).

Process of Compound [I—3] → Compound [I—4]

The desired compound is obtained in the same manner as described in the process of Compound [III] → Compound [II].

Process of Compound [I—4] → Compound [I—5]

The desired compound is obtained in the same manner as described in the process of Compound [II] → Compound [I—1].

Process of Compound [I—5] → Compound [I—2]

Compound [I—2] is obtained by subjecting Compound [I—5] to retro Michael reaction in the presence of an acid or an base in an inert solvent.

As the acid, an inorganic acid such as perchloric acid, etc. may be used. As the base, a tertiary amine such as N,N-dimethylaniline, etc. may be used. As the solvent, ethyl ether, tetrahydrofuran, ethylene glycol dimethyl ether, n-hexane, petroleum ether, benzene, N,N-dimethylformamide, ethyl acetate, acetone, methylene chloride, chloroform, dimethylsulfoxide, etc. may be used.

The acid or base is used in a molar amount of 1 to 100 times that of Compound [I—5].

The reaction is preferably carried out at 0 to 80°C and usually completed in 10 minutes to several hours.

Retro Michael reaction is disclosed in Tetrahedron Letters, No. 49, 4295 (1977), Synthesis, No. 12, 745 (1973).

Process of Compound [I—4] → Compound [I—6]

The desired compound is obtained in the same manner as described in the process of Compound [I—5] → Compound [I—2].

Process of Compound [I—6] → Compound [I—2]

The desired compound is obtained in the same manner as described in the process of Compound [II] → Compound [I—1].

Process of Compound [VI] → Compound [I—7]

A compound of formula [VI] wherein $X_a$ is methoxy is mitomycin B and a compound wherein $X_a$ is amino is mitomycin D. Though mitomycin D is obtained by fermentation as described before, the compound is also obtained by synthesis from mitomycin B (U.S. Patent No. 3,514,452).

Compound [I—7] is obtained by eliminating carbamic acid from Compound [VI] in the presence of a base in an innert solvent.

As the base, sodium carbonate, sodium hydroxide, sodium hydride, lithium hydride, triethylamine, potassium t-butoxide, 1,5-diazabicyclo[5.4.0]undecene-5, etc. may be used. As the solvent, tetrahydrofuran, dioxane, n-hexane, benzene, N,N-dimethylformamide, ethyl acetate, acetone, chloroform, etc. may be used.

The base is used in an amount of 1 to 30 times, preferably 1 to 10 times moles of a starting compound.

The reaction is carried out at −80 to 70°C and usually completed in 3 hours to 3 days.

Process of Compound [I—7] → Compound [I—8]

Compound [I—8] is obtained by alkylating Compound [I—7] with an alkylating agent in the presence of a base in an innert solvent.

As the alkylating agent, ethyl iodide, dimethyl sulfate, etc. may be used. As the base, sodium carbonate, sodium hydroxide, sodium hydride, lithium hydride, triethylamine, potassium t-butoxide, 1,5-diazabicyclo[5.4.0]undecene-5, etc. may be used. As the solvent, tetrahydrofuran, dioxane, n-hexane, benzene, N,N-dimethylformamide, ethyl acetate, acetone, chloroform, etc. may be used.

The alkylating agent and the base is respectively used in an amount of 1 to 50 times, preferably 1 to 10 times, moles of a starting compound.

The reaction is carried out at −30 to 50°C usually at room temperature and is completed in a few seconds to 24 hours.

Process of Compound [IX] → Compound [VIII]

In Compound [IX], a compound wherein $X_a$ is amino and the bond between 9-position and 10-position is represented by



is porfiromycin and a compound wherein $X_a$ is amino and the bond between 9-position and 10-position is represented by

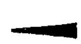

is mitomycin E.

The desired compound is obtained in the same manner as described in the process of Compound [V] → Compound [IV].

Process of Compound [VIII] → Compound [VII]

The desired compound is obtained in the same manner as described in the process of Compound [III] → Compound [II].

Process of Compound [VII] → Compound [I—8—1]

The desired compound is obtained in the same manner as described in the process of Compound [II] → Compound [I—1]

Process of Compound [I—9] → Compound [I—10]

Compound [I—9] partially overlaps with Compounds [I—2], [I—7] and [I—8] in the definition.

Compound [I—10] is obtained by hydrolyzing Compound [I—9] in an aqueous solution of a base.

As the base, inorganic bases such as sodium hydroxide, sodium carbonate, etc. and organic bases such as triethylamine, etc. may be used.

The base is usually used in a concentration of 0.01 to 1 normality.

The reaction is usually carried out at room temperature and completed in 30 minutes to several hours.

Process of Compound [I—10] → Compound [I—11]

Compound [I—11] is obtained by reacting Compound [I—10] with an alkylating agent in an innert solvent.

As the alkylating agent, diazoalkane such as diazomethane, etc., alkyl halide such as methyl iodide, etc., dialkyl sulfate such as dimethyl sulfate, etc. may be used. As the solvent, ethyl acetate, ethyl ether, etc. may be used. When an acid is produced by the reaction, the reaction is carried out in the presence of an acid acceptor such as potassium carbonate, triethylamine, etc.

Process of Compound [I—11] → Compound [I—9]

Compound [I—9] is obtained by reacting Compound [I—11] with ammonia or amine in an innert solvent such as methanol, etc.

Ammonia or amine is used in an amount of 1 to 100 times moles of Compound [I—11].

The reaction is usually carried out at 20 to 80°C and completed in 1 to several hours.

In each of the above processes, recovery of the desired compound from the reaction mixture after the reaction is carried out in a conventional method such as those described in the corresponding Example and Reference Example.

Fermentative production of Compounds 9 and 12

Compounds 9 and 12 are obtained by culturing the microorganism *Streptomyces caespitosus*, which is capable of producing these compounds, in a nutrient medium, accumulating one or more of the compounds in the culture liquor and recovering the same therefrom.

As the nutrient medium, any medium may be used so long as it properly contains carbon source, nitrogen source, inorganic materials and other nutrient according to the necessity for the strain. As the carbon source, glucose, fructose, blackstrap molasses, etc. may be used. As the nitrogen source, ammonia, ammonium phosphate, ammonium sulfate, ammonium acetate, urea, peptone, corn steep liquor, yeast extract, meat extract, dry yeast, etc. may be used. As the inorganic materials, potassium hydrogen phosphate, sodium chloride, calcium carbonate, etc. may be used.

Culturing is carried out with shaking or by a submerged stirring culturing method. Culturing temperature is usually at 25 to 35°C. PH during the culturing is preferably about 6 to 8 but is usually unnecessary to be controlled. The culturing is completed usually in 4 to 5 days.

After the completion of the culturing, recovery of the desired compound from the culture liquor may be carried out according to a method used usually for purification of an antibiotic, for example according to a method described in Examples 20 to 22.

Now, the present invention is further illustrated by the following representative examples and reference examples.

Example 1 (Compound [II] → [I—1])

38 mg of 1a-N-acetyl-10-decarbamoyl-10-methanesulfonyl-mitomycin C obtained in Reference Example 3 is dissolved in 8 ml of ethylene glycol dimethyl ether. 150 mg of 1,5-diazabicyclo[5.4.0]-undecene-5 is added to the solution and the mixture is refluxed with heating for 2 hours in an atmosphere of nitrogen. The reaction mixture is poured in saturated aqueous solution of sodium hydrogen carbonate and the mixture is extracted with ethyl acetate. The extract is washed with water, dried with anhydrous sodium sulfate and concentrated under reduced pressure to remove the solvent. The residue is purified by silica gel column chromatography using a mixed solvent of chloroform and acetone (6:4) (volume ratio; this is the same hereinafter) as a developer to obtain 24 mg of deep green crystals.

Physical properties of the substance are as follows.

Mass spectrum:

The substance exhibits molecular ion peak at m/e 315.

$^1$HNMR spectrum (in pyridine-$g_5$, δppm):

2.03(s,3H), 2.05(s,3H), 3.17(s,3H), 3.57(dd,1H), 3.64(dd,1H), 3.78(d,1H), 4.84(d,1H), 5.61(d,1H), 6.55(d,1H), 7.76(bs,2H)

IR spectrum (KBr tablet, $cm^{-1}$):

3435(w), 3330(m) (N—H stretch), 1695(s), 1594(vs), 1537(vs) (C=O stretch), 1656(m) (C=C stretch)

From the above properties, the substance is identified as 1a-N-acetyl-10-decarbamoyloxy-9-dehydro-mitomycin C. Yield 82%.

Example 2 (Compound [I—1] → [I—2])

57 mg of 1a-N-acetyl-10-decarbamoyloxy-9-dehydro-mitomycin C is dissolved in 1 ml of methanol. 1 ml of 10% aqueous solution of sodium hydrogen carbonate is added to the solution and the mixture is stirred at room temperature for 6 hours. After the completion of the reaction, the reaction mixture is extracted with ethyl acetate. The extract is dried with anhydrous sodium sulfate and concentrated under reduced pressure.

The residue is purified by silica gel column chromatography using a mixed solvent of chloroform and acetone (1:1) to obtain 36 mg of deep green crystals.

Physical properties of the substance are as follows.

Mass spectrum:

The substance exhibits molecular ion peak at m/e 273. Molecular weight obtained by high resolution mass spectrometry is 273.1130 (273.1113 as calculated).

$^1$HNMR spectrum (in pyridine-$d_5$, δppm):

2.00(s,3H), 2.77(dd,1H), 3.04(d,1H), 3.17(s,3H), 3.59(dd,1H), 4.67(d,1H), 5.50(d,1H), 6.50(d,1H), 7.57(bs,2H)

IR spectrum (KBr tablet, $cm^{-1}$):

3420(m) (N—H stretch), 3320(m) (N—H stretch), 3285(m) (N—H stretch), 1651(m) (C=C stretch), 1592(vs) (C=O stretch), 1532(vs) (C=O stretch)

Above IR spectrum is also shown in Fig. 5.

From the above properties, the substance is identified as 10-decarbamoyloxy-9-dehydro-mitomycin C. Yield 73%.

Example 3 (Compound [IV] → [I—3])

66 mg of 10-decarbamoyloxy-mitomycin C obtained in Reference Example 1 is dissolved in 5 ml of methylene chloride. 0.3 ml of acrolein (purity 90%) is added to the solution. The mixture is stirred at room temperature for 4 days and concentrated under reduced pressure. The residue is purified by silica gel column chromatography using a mixed solvent of chloroform and methanol (93:7) to obtain 76 mg of purplish black crystals.

Physical properties of the substance are as follows.

$^1$HNMR spectrum (in pyridine-$d_5$, δppm):

2.00(s,3H), 2.39—2.96(m,4H), 2.40(dd,1H), 2.91(d,1H), 3.22(s,3H), 3.61(dd,1H), 3.83(dd,1H), 4.34(dd,1H), 4.49(d,1H), 4.75(dd,1H), 6.18(bs,1H), 7.58(bs,2H), 9.76(t,1H)

IR spectrum (KBr tablet, $cm^{-1}$):

3430(m) (N—H stretch), 3335(m) (N—H stretch), 1722(m) (C=O stretch), 1604(s) (C=O stretch), 1554(vs) (C=O stretch)

From the above properties, the substance is identified as 10-decarbamoyl-1a-N-(2-formylethyl)-mitomycin C. Yield 97%.

Example 4 (Compound [I—3] → [I—4])

62 mg of 10-decarbamoyl-1a-N-(2-formylethyl)-mitomycin C obtained in Example 3 is dissolved in 0.33 ml of anhydrous pyridine. 15 μl of methanesulfonyl chloride is added to the solution in an atmosphere of nitrogen and the mixture is stirred under cooling with ice and sodium chloride for 2 hours. The reaction mixture is poured in a saturated aqueous solution of sodium hydrogen carbonate and the mixture is extracted with chloroform. The extract is washed with water, dried with anhydrous sodium sulfate and concentrated under reduced pressure. The residue is purified by silica gel column chromatography using a mixed solvent of chloroform and acetone (6:4) to obtain 44 mg of brownish black crystals.

Physical properties of the substance are as follows.

$^1$HNMR spectrum (in pyridine-$d_5$, δppm):

1.99(s,3H), 2.17—2.35(m,2H), 2.46(dd,1H), 2.64(dt,2H), 2.84(d,1H), 3.20(s,3H), 3.35(s,3H), 3.59(dd,1H), 4.01(dd,1H), 4.47(d,1H), 4.88(dd,1H), 5.37(dd,1H), 7.66(bs,2H), 9.75(t,1H)

IR spectrum (KBr tablet, $cm^{-1}$):

3440(m) (N—H stretch), 3340(m) (N—H stretch), 1720(m) (C=O stretch), 1604(vs) (C=O stretch), 1555(vs) (C=O stretch), 1351(vs) (antisym. $SO_2$, stretch), 1174(vs) (sym. $SO_2$ stretch)

From the above properties, the substance is identified as 10-decarbamoyl-1a-N-(2-formylethyl)-10-methanesulfonyl-mitomycin C. Yield 58%.

Example 5 (Compound [I—4] → [I—5])

52 mg of 10-decarbamoyl-1a-N-(2-formylethyl)-10-methanesulfonyl-mitomycin C obtained in Example 4 is dissolved in 10 ml of anhydrous ethylene glycol dimethyl ether. 210 mg of 1,5-diazabicyclo[5.4.0]undecene-5 is added to the solution and the mixture is refluxed in an atmosphere of nitrogen for 2 hours. The reaction mixture is poured in a saturated aqueous solution of sodium hydrogen carbonate and the mixture is extracted with ethyl acetate. The extract is washed with water, dried with anhydrous sodium sulfate and concentrated under reduced pressure. The residue is purified by silica gel column chromatography using a mixed solvent of chloroform and acetone (6:4) to obtain 16 mg of deep green crystals.

Physical properties of the substance are as follows.

$^1$HNMR spectrum (in pyridine-$d_5$, δppm):

2.00(s,3H), 2.29—2.78(m,4H), 2.40(dd,1H), 2.66(d,1H), 3.14(s,3H), 3.53(dd,1H), 4.64(d,1H), 5.51(d,1H), 6.51(d,1H), 7.68(bs,2H), 9.71(t,1H)

IR spectrum (KBr tablet, $cm^{-1}$):

3420(m) (N—H stretch), 3225(m) (N—H stretch), 1718(m) (C=O stretch), 1649(m) (C=C stretch), 1592(vs) (C=O stretch), 1535(vs) (C=O stretch)

From the above properties, the substance is identified as 10-decarbamoyloxy-9-dehydro-1a-N-(2-formylethyl)-mitomicin C. Yield 40%.

Example 6 (Compound [I—5] → [I—2])

26 mg of 10-decarbamoyloxy-9-dehydro-1a-N-(2-formylethyl)-mitomycin C obtained in Example 5 is dissolved in 4 ml of anhydrous methylene chloride. 100 mg of a salt of N,N-dimethylaniline with perchloric acid and subsequently 0.4 ml of N,N-dimethylaniline are added to the solution. The mixture is stirred at room temperature for 15 minutes and poured in a saturated aqueous solution of sodium hydrogen carbonate. The mixture is extracted with chloroform. The extract is washed with water, dried with anhydrous sodium sulfate and concentrated under reduced pressure. The residue is purified by silica gel column chromatography using a mixed solvent of chloroform and acetone (1:1) to obtain 15 mg of deep green crystals.

Mass spectrum, $^1$HNMR spectrum and IR spectrum of the substance are in accord with those of the desired compound in Example 2. Therefore, the substance is identified as 10-decarbamoyloxy-9-dehydro-mitomycin C. Yield 70%.

Example 7 (Compound [I—4] → [I—6])

33 mg of 10-decarbamoyloxy-1a-N-(2-formylethyl)-10-methanesulfonyl-mitomycin C obtained in Example 4 is dissolved in 5 ml of anhydrous methylene chloride. 120 mg of a salt of N,N-dimethylaniline with perchloric acid and subsequently 0.5 ml of N,N-dimethylaniline are added to the solution. The mixture is stirred at room temperature for 30 minutes. The reaction mixture is poured in a saturated aqueous solution of sodium hydrogen carbonate and the mixture is extracted with chloroform. The extract is washed with water, dried with anhydrous sodium sulfate and concentrated under reduced pressure. The residue is purified by silica gel column chromatography using a mixed solvent of chloroform and methanol (97:3) to obtain 21 mg of purplish red crystals.

Physical properties of the substance are as follows.

$^1$HNMR spectrum (in pyridine-$d_5$, δppm):

2.04(s,3H), 2.82(dd,1H), 3.22(s,3H), 3.23(d,1H), 3.63(dd,1H), 4.02(dd,1H), 4.52(d,1H), 5.18(dd,1H), 5.32(dd,1H), 5.57(bs,2H)

IR spectrum (KBr tablet, $cm^{-1}$):

3440(w) (N—H stretch), 3315(m) (N—H stretch), 1604(vs) (C=O stretch), 1555(vs) (C=O stretch), 1352(vs) (antisym. $SO_2$, stretch), 1174(vs) (sym. $SO_2$, stretch)

From the above properties, the substance is identified as 10-decarbamoyl-10-methanesulfonyl-mitomycin C. Yield 72%.

Example 8 (Compound [I—6] → [I—2])

20.5 mg of 10-decarbamoyl-10-methanesulfonyl-mitomycin C obtained in Example 7 is dissolved in 0.3 ml of anhydrous tetrahydrofuran. 30 mg of 1,5-diazabicyclo[5.4.0]undecene-5 is added to the

solution and the mixture is stirred at room temperature for 40 hours. The reaction mixture is poured in a saturated aqueous solution of sodium hydrogen carbonate and the mixture is extracted with ethyl acetate. The extract is washed with water, dried with anhydrous sodium sulfate and concentrated under reduced pressure. The residue is purified by silica gel column chromatography using a mixed solvent of chloroform and acetone (1:1) to obtain 1.2 mg of deep green crystals.

Mass spectrum, $^1$HNMR spectrum and IR spectrum of the substance are in accord with those of the desired compound of Example 2. Therefore, the substance is identified as 10-decarbamoyloxy-9-dehydro-mitomycin C. Yield 7.9%.

Example 9 (Compound [VI] → [I—7])

100 mg of mitomycin B is dissolved in 5 ml of dioxane. To the solution is added 100 mg of potassium t-butoxide and the mixture is stirred at room temperature for 2 days. The reaction mixture is neutralised with an excess amount of dry ice and subjected to filtration and the filtrate is concentrated under reduced pressure. The residue is subjected to silica gel column chromatography using a mixed solvent of acetone and chloroform (1:4) as a developer. Fractions of eluate which has a high Rf value and is blue are combined and concentrated under reduced pressure. The residue is crystallized from acetone and petroleum ether to obtain 14 mg of purplish black needle crystals.

Physical properties of the substance are as follows.

Mass spectrum:

The substance exhibits molecular ion peak of $M^+$=288.1086 (calculated molecular weight is 288.1110 as $C_{15}H_{16}N_2O_4$) by high resolution mass spectrometry.

$^1$HNMR spectrum (in $CDCl_3$, δppm):

6.32(s,1H), 5.53(s,1H), 3.00—4.20(1H), 2.27(2H), 2.18(s,3H), 3.98(d,1H), 3.45(d1H), 1.75(s,3H), 4.05(s,3H)

IR spectrum:

IR spectrum (KBr tablet) is shown in Fig. 1.

Rf value by TLC:

The substance exhibits a purplish blue single spot of Rf value of 0.70 by silica gel thin layer chromatography (Art 5719 made by Merck & Co., Inc.) using a mixed solvent of acetone and chloroform (1:1) as a developer (Mitomycin B exhibits Rf value of 0.30 in the same condition).

From the above properties, the substance is identified as 10-decarbamoyloxy-9-dehydro-mitomycin B. Yield 17.0%.

Example 10

1 g of 7-amino-7-demethoxy-mitomycin B and 3 g of silica gel (#7729 made by German Merck Co., Inc.) are added to 50 ml of tetrahydrofuran. To the mixture is added 480 mg of sodium hydride (containing 50% of oil) with stirring and the mixture is stirred at room temperature for 2 days. To the reaction mixture is added an excess amount of ethyl acetate saturated with water to decompose unreacted sodium hydride. An excess amount of dry ice is added to the mixture to neutralize the same. Then the mixture is subjected to filtration. The filtrate is concentrated under reduced pressure and the residue is subjected to silica gel column chromatography using a mixed solvent of acetone and chloroform (1:4) as a developer. Fractions of eluate which is eluted before unreacted starting material and is dark purplish green are combined and concentrated under reduced pressure to obtain 350 mg dark green needle crystals.

Physical properties of the substance are as follows.

Mass spectrum:

The substance exhibits molecular ion peak of $M^+$=273.1118 (calculated molecular weight is 273.1113 as $C_{14}H_{15}N_3O_3$) by high resolution mass spectrometry.

$^1$HNMR spectrum (in a mixed solvent of $CD_3OD$) and dimethyl sulfoxide-$D_6$, δppm):

5.90(s,1H), 5.32(s,1H), 6.33(s,1H), 2.23(2H), 2.17(s,3H), 4.22(d,1H), 3.43(d,1H), 1.70(s,3H), 6.47(s,2H)

IR spectrum:

IR spectrum (KBr tablet) is shown in Fig. 2.

Rf value by TLC (thin layer chromatography:

The substance exhibits a yellowish green single spot of Rf value of 0.42 by the same silica gel thin layer chromatography as described in Example 9 (7-amino-7-demethoxy-mitomycin B exhibits Rf value of 0.10 in the same condition).

From the above properties, the substance is identified as 7-amino-10-decarbamoyloxy-9-dehydro-7-demethoxy-mitomycin B. Yield 42.8%.

Example 11 (Compound [I—7] → [I—8])

20 mg of 10-decarbamoyloxy-9-dehydro-mitomycin B obtained in Example 9 is added to a mixed solvent of 0.3 ml of dimethylformamide and 1 ml of benzene. To the mixture is added 20 mg of sodium

hydride (containing 50% of oil) with stirring. 0.035 ml of dimethyl sulfate is added to the mixture and the mixture is stirred for 2 minutes. Ethyl acetate saturated with water is added to the mixture to decompose unreacted sodium hydride. The mixture is filtered and to the filtrate is added 10 ml of ethyl acetate. The mixture is washed 5 times with 2 ml of water. The organic layer is dried with anhydrous sodium sulfate and concentrated under reduced pressure. The residue is subjected to silica gel column chromatography using a mixed solvent of acetone and chloroform (1:9) to obtain 8.7 mg of purplish blue needle crystals.

Physical properties of the compound are as follows.

Mass spectrum:

The substance exhibits molecular ion peak of $M^+$=302.1280 (calculated molecular weight is 302.1266 as $C_{16}H_{18}N_2O_4$).

$^1$HNMR spectrum (in $CDCl_3$, $\delta$ppm):

6.32(s,1H), 5.50(s,1H), 3.07(s,3H), 2.25(2H), 2.22(s,3H), 4.08(d1H), 3.41(dd,1H), 1.85(s,3H), 4.08(s,3H)

IR spectrum:

IR spectrum (KBr tablet) is shown in Fig. 3.

Rf value by TLC:

The substance exhibits a purplish blue single spot of Rf value of 0.80 by the same silica gel thin layer chromatography as described in Example 9 (10-decarbamoyloxy-9-dehydro-mitomycin B exhibits Rf value of 0.70 in the same condition).

From the above properties, the substance is identified as 9a-O-methyl-10-decarbamoyloxy-9-dehydro-mitomycin B. Yield 41.5%.

Example 12 (Compound [VII] → [I—8—1])

17 mg of 10-decarbamoyl-10-methanesulfonyl-porfiromycin obtained in Reference Example 6 is dissolved in 1 ml of anhydrous tetrahydrofuran. 54 mg of 1,5-diazabicyclo[5.4.0]undecene-5 is added to the solution and the mixture is refluxed in an atmosphere of nitrogen for 5 hours. The reaction mixture is concentrated under reduced pressure and the residue is purified by silica gel column chromatography using a mixed solvent of chloroform and acetone (4:1) to obtain 7.5 mg of purplish blue crystals.

Physical properties of the substance are as follows.

Mass spectrum:

The substance exhibits molecular ion peak at m/e 287.

$^1$HNMR spectrum (in $CD_3OD$, $\delta$ppm):

1.78(s,3H), 2.21(s,3H), 2.44(bs,2H), 3.06(s,3H), 3.42(dd,1H), 4.26(d,1H), 5.34(d,1H), 6.08(d,1H)

From the above properties, the substance is identified as 10-decarbamoyloxy-9-dehydro-porfiromycin. Yield 58.9%.

Example 13 (Compound [VII] → [I—8—1])

31.5 mg of 10-decarbamoyl-10-methanesulfonyl-mitomycin E obtained in Reference Example 7 is dissolved in 1 ml of anhydrous tetrahydrofuran. 70 mg of 1,5-diazabicyclo[5.4.0]undecene-5 is added to the solution and the mixture is refluxed in an atmosphere of nitrogen for 7 hours. After the reaction, the reaction mixture is concentrated under pressure and the residue is purified by silica gel column chromatography using a mixed solvent of chloroform and acetone (4:1) to obtain 15.3 mg of purplish blue crystals.

Mass spectrum and $^1$HNMR spectrum of the substance are in accord with those of the desired compound of Example 12. Therefore, the substance is identified as 10-decarbamoyloxy-9-dehydro-porfiromycin. Yield 64.8%.

Example 14 (Compound [I—9] → [I—10])

18 mg of 10-decarbamoyloxy-9-dehydro-mitomycin C obtained in Example 2 is dissolved in 3.75 ml of 0.1N sodium hydroxide. The solution is stirred at room temperature for 45 minutes. The reaction solution is adjusted to pH 4 with diluted hydrochloric acid and extracted with ethyl acetate. The extract is washed with water, dried with anhydrous sodium sulfate and concentrated under reduced pressure. The residue is purified by silica gel column chromatography using a mixed solvent of chloroform and methanol (9:1) to obtain 12 mg of purplish black crystals.

Physical properties of the substance are as follows.

Mass spectrum:

The substance exhibits molecular ion peak at m/e 274.

$^1$HNMR spectrum (in pyridine-$d_5$, $\delta$ppm):

2.05(s,3H), 2.80(dd,1H), 3.08(d,1H), 3.19(s,3H), 3.58(dd,1H), 4.50(d,1H), 5.55(d,1H), 6.52(d,1H)

IR spectrum (KBr tablet, $cm^{-1}$):

3285(w) (N—H stretch), 1644(vs) (C=C stretch), 1630(vs) (C=O stretch), 1548(vs) (C=O stretch) (Fig. 6)

From the above properties, the substance is identified as 7-deamino-10-decarbamoyloxy-9-dehydro-7-hydroxy-mitomycin C. Yield 66%.

Example 15 (Compound [I—9] → [I—10])

134 mg of 10-decarbamoyloxy-9-dehydro-porfiromycin obtained in Example 12 is dissolved in 25 ml of 0.1N sodium hydroxide. The solution is stirred at room temperature for 4 hours. The reaction solution is adjusted to pH 3 with diluted hydrochloric acid and extracted with ethyl acetate. The extract is washed with water, dried with anhydrous magnesium sulfate and concentrated under reduced pressure. The residue is purified by silica gel column chromatography using a mixed solvent of chloroform and methanol (9:1) and crystallized from a mixed solvent of chloroform and methanol to obtain 115 mg of dark green needle crystals.

Physical properties of the substance are as follows.

Mass spectrum:

The substance exhibits molecular ion peak at m/e 288.

$^1$HNMR spectrum (in pyridine-$d_5$, $\delta$ppm):

2.03(s,3H), 2.12(s,3H), 2.22(dd,1H), 2.48(d,1H), 3.13(s,3H), 3.47(dd,1H), 4.44(d,1H), 5.44(bs,1H), 6.48(bs,1H)

IR spectrum:

IR spectrum (KBr tablet) is shown in Fig. 8.

From the above properties, the substance is identified as 10-decarbamoyloxy-9-dehydro-7-deamino-7-hydroxy-porfiromycin. Yield 85.3%.

Example 16 (Compound [I—10] → [I—11])

12 mg of 7-deamino-10-decarbamoyloxy-9-dehydro-7-hydroxy-mitomycin C obtained in Example 14 is dissolved in 3 ml of ethyl acetate. An excess amount of ethyl ether solution of diazomethane is added dropwise to the solution under cooling with ice and the mixture is allowed to stand for 10 minutes. The mixture is concentrated under reduced pressure and the residue is purified by silica gel column chromatography using a mixed solvent of chloroform and acetone (6:4) to obtain 8 mg of purplish black crystals.

Physical properties of the substance are as follows.

Mass spectrum:

The substance exhibits molecular ion peak at m/e 288. Molecular weight obtained by high resolution mass spectrometry is 288.1120 (288.1110 as calculated).

$^1$HNMR spectrum (in pyridine-$d_5$, $\delta$ppm):

1.83(dd,1H), 3.07(d,1H), 3.16(s,3H), 3.53(dd,1H), 4.01(s,3H), 4.33(d,1H), 5.59(d,1H), 6.57(d,1H)

IR spectrum (KBr tablet, $cm^{-1}$):

3305(vw) (N—H stretch), 1650(vs) (C=O stretch), 1554(vs) (C=O stretch). Above IR spectrum is also shown in Fig. 7.

From the above properties, the substance is identified as 10-decarbamoyloxy-9-dehydro-mitomycin A. Yield 63%.

Example 17 (Compound [I—10] → [I—11])

27.4 mg of 10-decarbamoyloxy-9-dehydro-7-deamino-7-hydroxy-porfiromycin is dissolved in 4 ml of methanol. An excess amount of an ethyl ether solution of diazomethane is added dropwise to the solution under cooling with ice and the mixture is allowed to stand for 10 minutes. The reaction mixture is concentrated under reduced pressure and the residue is purified by silica gel column chromatography using a mixed solvent of chloroform and acetone (98:2) and crystallized from n-hexane to obtain 25.9 mg of purplish black needle crystals.

Physical properties of the substance are as follows.

Mass spectrum:

The substance exhibits molecular ion peak at m/e 302.

$^1$HNMR spectrum (in $CD_3OD$, $\delta$ppm):

1.85(s,3H), 2.21(s,3H), 2.46(bs,2H), 3.05(s,3H), 3.39(dd,1H), 4.03(s,3H), 4.07(d,1H), 5.45(d,1H), 6.22(d,1H)

IR spectrum (KBr tablet):

In accord with Fig. 3.

From the above properties, the substance is identified as 10-decarbamoyloxy-9-dehydro-7-deamino-7-methoxy-porfiromycin (that is, 9a-O-methyl-10-decarbamoyloxy-9-dehydro-mitomycin B). Yield 90.2%.

Example 18 (Compound [I—11] → [I—9])

5.2 mg of 10-decarbamoyloxy-9-dehydro-mitomycin A obtained in Example 16 is dissolved in 2 ml of methanol saturated with ammonia. The solution is stirred at room temperature for 2 hours. The solution is concentrated under reduced pressure and the residue is purified by silica gel column chromatography using a mixed solvent of chloroform and acetone (1:1) to obtain 4.1 mg of deep green crystals.

Mass spectrum, $^1$HNMR spectrum and IR spectrum of the substance are in accord with those of the desired compound of Example 2. Therefore, the substance is identified as 10-decarbamoyloxy-9-dehydro-mitomycin C. Yield 83%.

Example 19 (Compound [I—11] → [I—9])

10 mg of 9a-O-methyl-10-decarbamoyloxy-9-dehydro-mitomycin B obtained in Example 11 is added to 5 ml of methanol saturated with ammonia. The mixture is stirred at room temperature for 18 hours. The reaction mixture is concentrated to dryness under reduced pressure and the residue is crystallized from acetone and petroleum ether to obtain 6.2 mg of green needle crystals.

Physical properties of the substance are as follows.

Mass spectrum:

The substance exhibits molecular ion peak of $M^+=287.1252$ (calculated molecular weight is 287.1269 as $C_{15}H_{17}N_3O_3$) by high resolution mass spectrometry.

$^1$HNMR spectrum (in $CD_3OD$, $\delta$ppm):

6.08(d,1H), 5.34(d,1H), 3.06(s,3H), 2.43(2H), 2.21(s,3H) 4.26(d,1H), 3.43(dd,1H), 1.77(s,3H), 4.81(exchange with $CD_3OD$)

IR spectrum:

IR spectrum (KBr tablet) is shown in Fig. 4.

Rf value by TLC:

The substance exhibits a deep green single spot of Rf value of 0.63 by silica gel TLC (Art 5719, made by Merck & Co., Inc.) (9a-O-methyl-10-decarbamoyloxy-9-dehydro-9a-dehydroxy-mitomycin B exhibits Rf value of 0.80 in the same condition).

From the above properties, the substance is identified as 7-amino-9a-O-methyl-10-decarbamoyloxy-9-dehydro-7-demethoxy-mitomycin B (that is, 10-decarbamoyloxy-9-dehydro-porfiromycin). Yield 65.2%.

Example 20 (Compound [I—12] by fermentation)

(1) Culturing of *Streptomyces caespitosus* ATCC 27422

*Streptomyces caespitosus* ATCC 27422 is used as a seed strain. One loopful of the strain is inoculated in 50 ml of the following first seed medium in a 250 ml-Erlenmeyer flask. Culturing is carried out at 28°C for 2 days. The first seed culture is transferred to a 2 l-Erlenmeyer flask with baffles containing 500 ml of a second seed medium. Culturing is carried out at 28°C for 2 days. 1.5 l of the second seed culture (3 flasks) is transferred to a 200 l-culturing tank containing 100 l of a third seed medium. Culturing is carried out at 28°C for 2 days with aeration and stirring (revolution: 250 r.p.m., aeration: 60 l/min.).

First, second and third seed medium:

glucose 15 g/l, soluble starch 5 g/l, dry yeast 10 g/l, NaCl 5 g/l and $CaCO_3$ 3 g/l (pH 7.0 before sterilization at 120°C for 20 minutes).

100 l of the third culture is transferred to 2 Kl-fermentation tank containing 1 Kl of a fermentation medium.

Fermentation medium:

sucrose 15 g/l, soluble starch 20 g/l, soybean cake 40 g/l, NaCl 5 g/l, $CoCl_2 \cdot 6H_2O$ 200 mg/l and normal paraffin 5 ml/l (pH 7.2 before sterilization at 120°C for 20 minutes).

Culturing is carried out at 28°C for 5 days with aeration and stirring (revolution: 80 r.p.m., aeration: 400 l/min.).

(2) Isolation of the desired compound

After the culturing, 20 Kg of sodium tetraborate ($Na_2B_4O_7 \cdot 10H_2O$) is dissolved in the culture liquor and 100 Kg of Radiolite #600 (trade mark of a filtrate aid, made by Showa Kagaku Kogyo Co., Ltd., Japan), is added. The microbial cells are filtered off and the filtrate is passed through a column packed with 50 l of Diaion HP—20 (trade mark for an ion exchange resin, made by Mitsubishi Kasei Kogyo, Co., Ltd., Japan). The resin is washed with 250 l of deionization water and elution is carried out with 250 l of 50% aqueous methanol and subsequently with 150 l of methanol. 200 l of the eluate containing the desired compound is concentrated under reduced pressure to about 26 l. About 8 Kg of sodium chloride is dissolved in the concentrate. The concentrate is extracted 5 times respectively with 17 l of chloroform and the chloroform layers are combined and concentrated to 1 l. To the concentrated

solution is added anhydrous sodium sulfate for dehydration. The solution is passed through a column packed with 7 l of silica gel. Elution is carried out with a mixed solvent of chloroform and methanol (100:1—5). Fractions of the eluate containing the desired compound are combined, concentrated and subjected to silica gel column chromatography using the same solvent system as above. Fractions containing the desired compound are concentrated and subjected to silica gel column chromatography using a mixed solvent of ethyl acetate and acetone (100:1). (This procedure is once repeated). Fractions containing the desired product are concentrated and subjected to alumina column chromatography using a mixed solvent of chloroform and acetone (98:2). The eluate is concentrated to dryness and the residue is crystallized from acetone and petroleum ether to obtain 1.9 mg of green needle crystals.

In the above isolation process, "the desired compound" is pursued by bioassay using *Bacillus subtilis* ATCC 6633 as the test microorganism and/or silica gel TLC using a mixed solvent of chloroform and acetone (6:4) as developer (this is same in Examples 21 and 22).

Physical properties of the substance are as follows.

Mass spectrum:
Found 287.1252 (Calculated as $C_{15}H_{17}N_3O_3$ 287.1269)
$^1$HNMR spectrum (in $CD_3OD$, $\delta$ppm):
1.77(s,3H), 2.21(s,3H), 2.44(bs,2H), 3.06(s,3H), 3.42(dd,1H), 4.26(d,1H), 5.34(d,1H), 6.07(d,1H)
IR spectrum:
IR spectrum (KBr tablet) accords with Fig. 4.
Rf value by TLC:

TABLE 6

TLC by silica gel (art 5714 made by Merck & Co., Inc.)

| Antibiotic | Developer: Chloroform: Methanol (9:1) | Developer: Ethyl acetate: Acetone (6:4) | Developer: Chloroform: Acetone (6:4) |
|---|---|---|---|
| Mitomycin A | 0.40 | 0.46 | 0.14 |
| ,, B | 0.31 | 0.48 | 0.19 |
| ,, C | 0.21 | 0.24 | 0.05 |
| ,, D | 0.14 | 0.24 | 0.05 |
| ,, E | 0.30 | 0.33 | 0.11 |
| Porfiromycin | 0.36 | 0.48 | 0.16 |
| Compound 12 | 0.74 | 0.77 | 0.60 |
| ,, 9 | 0.70 | 0.79 | 0.67 |
| ,, 10 | 0.93 | 0.82 | 0.77 |

TABLE 7

TLC by alumina (art 5731, made by Merck & Co., Inc.)

| Antibiotic | Developer | | |
|---|---|---|---|
| | Chloroform: Methanol (9 : 1) | Ethyl acetate: Acetone (6 : 4) | Chloroform: Acetone (6 : 4) |
| Mitomycin A | 0.56 | 0.21 | 0.08 |
| ,, B | 0.40 | 0.14 | 0.04 |
| ,, C | 0.28 | 0.09 | 0.02 |
| ,, D | 0.12 | 0.05 | 0.01 |
| ,, E | 0.46 | 0.21 | 0.07 |
| Porfiromycin | 0.47 | 0.29 | 0.08 |
| Compound 12 | 0.75 | 0.76 | 0.67 |
| ,, 9 | 0.72 | 0.73 | 0.58 |
| ,, 10 | 0.83 | 0.84 | 0.84 |

Melting point:
about 270°C (browning at about 220°C)
Electronic absorption spectrum (in MeOH)
222 nm (log $\varepsilon$ 4.02), 289(4.03), 373(4.25), 602(2.37)
Elementary analysis (%):

| | H | C | N |
|---|---|---|---|
| Found | 5.95 | 62.73 | 14.34 |
| Calculated as $C_{15}H_{17}N_3O_3$ | 5.96 | 62.70 | 14.63 |

Specific rotation:
Measurement is impossible as the substance is deep green.
Distinction of basic, acidic or neutral property:
Neutral
Solubility:
Soluble in methanol, ethanol, acetone, ethyl acetate and chloroform, very slightly soluble in benzene, ethyl ether and water, insoluble in n-hexane

From the above physical properties, the substance is identified as 7-amino-9a-O-methyl-10-decarbamoyloxy-9-dehydro-7-demethoxy-mitomycin B (that is, 10-decarbamoyloxy-9-dehydro-porfiromycin).

Example 21 (Compound [I—12] by fermentation)

(1) Culturing of *Streptomyces caespitosus* ATCC 27422

The same seed media (first, second and third media) and fermentation medium as described in Example 20 are used.

One loopful of the strain is inoculated in 50 ml of the seed medium in a 250 ml-Erlenmeyer flask and culturing is carried out at 28°C for 2 days. Second and third culturings are carried out in the same manner as described in Example 20. Whole amount of the third culture is transferred in a 2 Kl-fermentation tank containing 1 Kl of a fermentation medium and main fermentation is carried out in the same manner as described in Example 20.

(2) Isolation of the desired compound

After the completion of culturing, a concentrated extract with chloroform is obtained in the same manner as described in Example 20. The extract is dehydrated with anhydrous sodium sulfate and passed through a column packed with alumina. Elution is carried out with chloroform. Fractions containing the desired compound are combined and concentrated. The concentrate is allowed to stand

overnight in a refrigerator. The precipitate is filtered off and the filtrate is concentrated under reduced pressure. A small amount of chloroform is added to the residue and insoluble materials are filtered off. The filtrate is subjected to silica gel column chromatography using a mixed solvent of chloroform and acetone (10:0—3). Fractions containing the desired compound are combined and concentrated. The concentrate is again subjected to the same silica gel column chromatography as above. Fractions containing the desired compound are combined and concentrated. The concentrate is crystallized from acetone to obtain 5.6 mg of purplish black needle crystals.

Physical properties of the compound are as follows.

Mass spectrum:

Found 288.1135 (calculated as $C_{15}H_{16}N_2O_4$ 288.1110)

$^1$HNMR spectrum (in $CD_3OD$, $\delta$ppm):

1.84(s,3H), 2.21(s,3H), 2.44(bs,2H), 3.46(dd,1H), 4.02(s,3H), 4.03(d,1H), 5.48(d,1H), 6.09(d,1H)

IR spectrum:

IR spectrum (KBr tablet) accords with Fig. 1.

Rf value by TLC:

Rf value by TLC is shown in Tables 6 and 7.

Melting point:

about 125°C

Electronic absorption spectrum (in MeOH):

226 nm(log $\varepsilon$ 4.08), 2.91(4.03), $324^{sh}$(3.93) 578(3.04)

Elementary analysis (%):

| | H | C | N |
|---|---|---|---|
| Found | 5.64 | 62.34 | 9.37 |
| Calculated as $C_{15}H_{16}N_2O_4$ | 5.59 | 62.49 | 9.72 |

Specific rotation:

Measurement is impossible as the substance is purplish black.

Distinction of acidic, basic or neutral property:

Neutral

Solubility:

Soluble in methanol, ethanol, acetone, ethyl acetate and chloroform, very slightly soluble in benzene, ethyl ether and water, insoluble in n-hexane.

From the above properties, the compound is identified as 10-decarbamoyloxy-9-dehydro-mitomycin B.

Example 22 (Compound [I—12] by fermentation)

(1) Culturing of *Streptomyces caespitosus* ATCC 27422

The same seed media (first, second and third seed media) and fermentation medium as described in Example 20 are used. Culturing is also carried out in the same manner as described in Example 20.

(2) Isolation of the desired compound

After the culturing, a concentrated extract with chloroform is obtained in the same manner as described in Example 20. Anhydrous sodium sulfate is added to the extract for dehydration. The resultant extract is passed through a column packed with alumina. Elution is carried out with chloroform. Fractions containing the desired compound are combined and concentrated. The concentrate is allowed to stand overnight in a refrigerator. The precipitate is filtered off and the filtrate is concentrated under reduced pressure. A small amount of chloroform is added to the residue and insoluble materials are filtered off. The filtrate is subjected to silica gel column chromatography using a mixed solvent of chloroform and acetone (4:1 to 3:2). Fractions containing the desired compound are combined and concentrated. The concentrate is again subjected to silica gel column chromatography using a mixed solvent of chloroform and acetone (49:1 to 9:1). Fractions containing the desired compound are combined and concentrated. The concentrate is subjected to alumina column chromatography using a mixed solvent of bezene and ethyl acetate (95:5) as a developer. The filtrate is concentrated to dryness and the residue is crystallized from n-hexane to obtain 2.8 mg of purplish blue needle crystals.

Physical properties of the substance are as follows.

Mass spectrum:

Found 302.1279 (Calculated as $C_{16}H_{18}N_2O_4$ 302.1266)

$^1$HNMR spectrum (in $CD_3OD$, $\delta$ppm):

1.85(s,3H), 2.21(s,3H), 2.46(bs,2H), 3.05(s,3H), 3.38(dd,1H), 4.02(s,3H), 4.07(d,1H), 5.44(d,1H), 6.21(d,1H)

IR spectrum:
IR spectrum (KBr tablet) accords with Fig. 3.
Rf value by TLC:
Rf value by TLC is shown in Tables 6 and 7.
Melting point:
92 to 93°C
Electronic absorption spectrum (in MeOH):
220 nm(log $\varepsilon$ 4.15), 289(4.05), 320(4.00), 569(3.08)
Elementary analysis (%):

| | H | C | N |
|---|---|---|---|
| Found | 5.99 | 63.29 | 8.88 |
| Calculated as $C_{16}H_{18}N_2O_4$ | 6.00 | 63.56 | 9.27 |

Specific rotation:
Measurement is impossible as the substance is purplish blue.
Distinction of acidic, basic or neutral property:
Neutral
Solubility
Soluble in methanol, ethanol, acetone, ethyl acetate, chloroform, benzene, ethyl ether and n-hexane, very slightly soluble in water

From the above properties, the substance is identified as 9a-O-methyl-10-decarbamoyloxy-9-dehydro-mitomycin B.

Reference Example 1 (Compound [V] → [IV])

230 mg of sodium is dissolved in 50 ml of isopropanol and 500 mg of mitomycin C is added to the solution. The mixture is stirred at room temperature for 8 hours. The reaction mixture is neutralized with an excess amount of dry ice. The precipitate is filtered out and the filtrate is concentrated under reduced pressure. The residue is purified by silica gel column chromatography using a mixed solvent of chloroform and methanol (9:1) to obtain 270 mg of purplish black crystals.

The substance is identified as known 10-decarbamoyl-mitomycin C from melting point, $^1$HNMR spectrum, TLC, etc. Yield 62%.

Reference Example 2 (Compound [IV] → [III])

138 mg of 10-decarbamoyloxy-mitomycin C obtained in Reference Example 1 is dissolved in 1 ml of pyridine. 60 $\mu l$ of acetic anhydride is added to the solution under cooling with ice and sodium chloride and the mixture is stirred in an atmosphere of nitrogen for 1 hour. The reaction mixture is poured in a saturated aqueous solution of sodium hydrogen carbonate and the mixture is extracted with ethyl acetate. The extract is washed with water, dried with anhydrous sodium sulfate and concentrated under reduced pressure. The residue is purified by silica gel column chromatography using a mixed solvent of chloroform and methanol (95:5) to obtain 153 mg of purplish black crystals.

Physical properties of the substance are as follows.

Mass spectrum:
The substance exhibits molecular ion peak at m/e 333.
$^1$HNMR spectrum (in pyridine-$d_5$, $\delta$ppm):
2.05(s,3H), 2.23(s,3H), 3.23(s,3H), 3.57(dd,1H), 3.66(dd,1H), 3.94(dd,1H), 3.98(d,1H), 4.33(d,1H), 4.76(d,1H) 4.84(dd,1H), 7.57(bs,2H)
IR spectrum (KBr tablet, $cm^{-1}$):
3435(m), 3335(m) (N—H stretch), 1694(m), 1605(s), 1555(vs) (C=O stretch)

From the above properties, the substance is identified as 1a-N-acetyl-10-decarbamoyl-mitomycin C. Yield 97%.

Reference Example 3 (Compound [III] → [II])

62 mg of 1a-N-acetyl-10-decarbamoyl-mitomycin C obtained in Reference Example 2 is dissolved in 0.5 ml of anhydrous pyridine. 15 $\mu l$ of methanesulfonyl chloride is added to the solution in an atmosphere of nitrogen under cooling with ice and sodium chloride and the mixture is stirred for 2 hours. The reaction mixture is poured in a saturated aqueous solution of sodium hydrogen carbonate and the mixture is extracted with ethyl acetate. The extract is washed with water, dried with anhydrous sodium sulfate and concentrated under reduced pressure. The residue is purified by silica gel column chromatography using a mixed solvent of chloroform and methanol (96:4) to obtain 74 mg of purplish black crystals.

Physical properties of the substance are as follows.

$^1$HNMR spectrum (in pyridine-$d_5$, δppm):
2.04(s,3H), 2.11(s,3H), 3.20(s,3H), 3.46(s,3H), 3.60(dd,1H), 3.61(dd,1H), 3.79(d,1H), 4.09(dd,1H), 4.84(dd,1H), 5.58(dd,1H), 7.71(bs,2H)
$^1$HNMR spectrum (KBr tablet, $cm^{-1}$):
3445(w), 3235(m) (N—H stretch), 1697(s), 1605(s), 1563(vs) (C=O stretch), 1350(vs) (antisym. $SO_2$ stretch), 1173(vs) (sym. $SO_2$ stretch)
From the above properties, the substance is identified as 1a-N-acetyl-10-decarbamoyl-10-methanesulfonyl-mitomycin C. Yield 97%.

Reference Example 4 (Compound [IX] → [VIII])

500 mg of porfiromycin is added to 150 ml of isopropanol containing 1.5 g of sodium isopropoxide. The mixture is stirred at room temperature for 6 hours. The reaction mixture is neutralized with an excess amount of dry ice and the deposit is filtered out. The filtrate is concentrated under reduced pressure and the residue is purified by silica gel column chromatography using a mixed solvent of chloroform and methanol (94:6) to obtain 299 mg of purplish blue crystals.

Physical properties of the substance are as follows.

$^1$HNMR spectrum (in $CD_3OD$, δppm):
1.75(S,3H), 2.29(s,3H), 2.45(dd,1H), 2.54(d,1H), 3.20(s,3H), 3.34(dd,1H), 3.46(dd,1H), 4.09(dd,1H), 4.16(d,1H)
From the above properties, the substance is identified as 10-decarbamoyl-porfiromycin. Yield 68.2%.

Reference Example 5 (Compound [IX] → [VIII])

50 mg of mitomycin E (7-demethoxy-7-amino-9a-O-methyl-mitomycin B) is added to 6 ml of isopropanol containing 500 mg of sodium isopropoxide. The mixture is stirred at room temperature for 7 hours. The reaction mixture is neutralized with an excess amount of dry ice and the deposit is filtered out. The filtrate is concentrated under reduced pressure and the residue is purified by silica gel column chromatography using a mixed solvent of chloroform and methanol (95:5) to obtain 25.6 mg of purple crystals.

The substance exhibits molecular ion peak at m/e 305 in mass spectrum. Therefor, the substance is identified as 10-decarbamoyl-mitomycin E. Yield 58.3%.

Reference Example 6 (Compound [VIII] → [VII])

105,4 mg of 10-decarbamoyl-porfiromycin obtained in Reference Example 4 is dissolved in 2 ml of anhydrous pyridine. 0.05 ml of methanesulfonyl chloride is added to the solution and the mixture is stirred for 20 minutes. The reaction mixture is poured in 10 ml of a saturated aqueous solution of sodium hydrogen carbonate and the mixture is extracted with ethyl acetate. The extract is washed with water, dried with anhydrous sodium sulfate and concentrated under reduced pressure. The residue is purified by silica gel column chromatography using a mixed solvent of chloroform and methanol (95:5) to obtain 130.7 mg of purple solid.

Physical properties of the substance are as follows.

Mass spectrum:
The substance exhibits molecular ion peak at m/e 383.
$^1$HNMR spectrum (in $CD_3OD$, δppm):
1.74(s,3H), 2.33(s,3H), 2.45(dd,1H), 2.53(d,1H), 3.18(s,3H), 3.22(s,3H), 3.46(dd,1H), 3.62(dd,1H), 4.19(d,1H), 4.42(dd,1H), 4.82(dd,1H)
From the above properties, the substance is identified as 10-decarbamoyl-10-methanesulfonyl-porfiromycin. Yield 98.6%.

Reference Example 7 (Compound [VIII] → [VII])

25.6 mg of 10-decarbamoyl-mitomycin E obtained in Reference Example 5 is dissolved in 0.3 ml of anhydrous pyridine. 0.013 ml of methanesulfonyl chloride is added to the solution and the mixture is stirred at room temperature for one hour. The reaction mixture is poured in 10 ml of a saturated aqueous solution of sodium hydrogen carbonate and the mixture is extracted with ethyl acetate. The extract is treated in the same manner as described in Reference Example 6 to obtain 31.5 mg of bluish green crystals.

Physical properties of the substance are as follows:

Mass spectrum:
The substance exhibits molecular ion peak at m/e 383.
$^1$HNMR spectrum (in $CD_3OD$, δppm):
1.73(s,3H), 2.34(s,3H), 2.45(d,1H), 2.64(dd,1H), 3.10(s,3H), 3.38(s,3H), 3.64(dd,1H), 3.84(dd,1H), 3.93(d,1H), 4.50(dd,1H), 4.92(dd,1H)

From the above properties, the substance is identified as 10-decarbamoyl-10-methanesulfonyl-mitomycin E. Yield 98.0%.

**Claims**

1. A mitomycin represented by the general formula

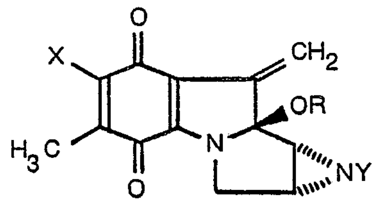

wherein
X is amino, hydroxy or alkoxy having 1—4 carbon atoms
Y is hydrogen, methyl, —$COR^1$ or —$CH_2CH_2Z$
$R_1$ is hydrogen, alkyl having 1—3 carbon atoms, chloromethyl, phenyl or p-methoxyphenyl
Z is alkoxycarbonyl having 2—5 carbon atoms, —$COR^4$ or cyano
$R^4$ is hydrogen or alkyl having 1—3 carbon atoms
R is hydrogen or alkyl having 1—4 carbon atoms.

2. A mitomycin of claim 1 represented by the general formula

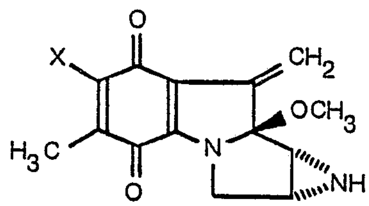

wherein X has the same meaning as defined above.

3. A mitomycin of claim 2 represented by the formula

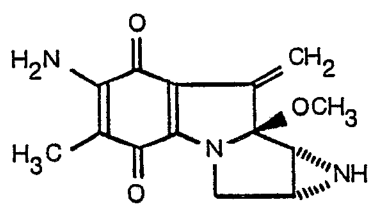 ,

that is 10-decarbamoyloxy-9-dehydro-mitomycin C.

4. A mitomycin of claim 2 represented by the formula

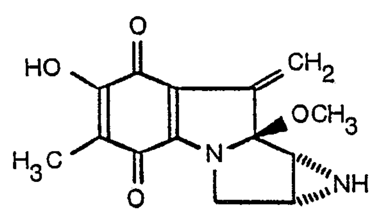 ,

that is 7-deamino-10-decarbamoyloxy-9-dehydro-7-hydroxy-mitomycin C.

5. A mitomycin of claim 2 represented by the formula

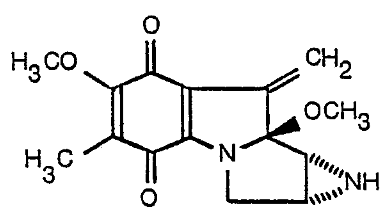 ,

that is 10-decarbamoyloxy-9-dehydro-mitomycin A.

6. A mitomycin of claim 1 represented by the general formula

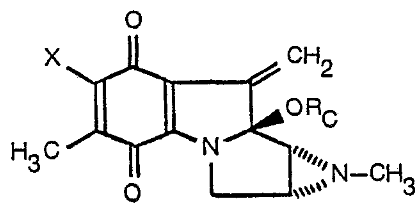

wherein X and $R_c$ have the same meaning as defined above.

7. A mitomycin of claim 6 represented by the formula

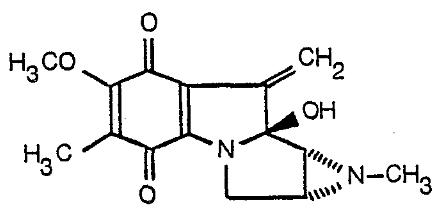,

that is 10-decarbamoyloxy-9-dehydro-mitomycin B.

8. A mitomycin of claim 6 represented by the formula

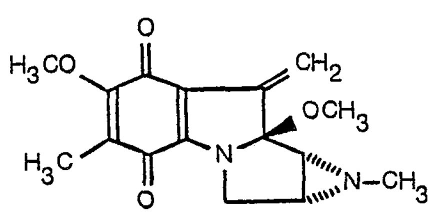,

that is 9a-O-methyl-10-decarbamoyloxy-9-dehydro-mitomycin B.

9. A mitomycin of claim 6 represented by the formula

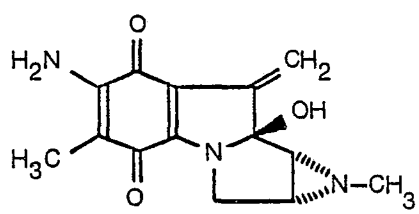,

that is 7-amino-10-decarbamoyloxy-9-dehydro-7-demethoxy-mitomycin B.

10. A mitomycin of claim 6 represented by the formula

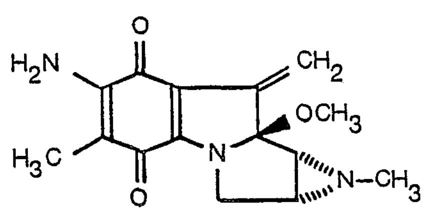,

that is 10-decarbamoyloxy-9-dehydro-porfiromycin.

11. A mitomycin of claim 6 represented by the formula

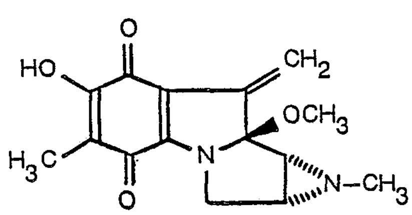,

that is 10-decarbamoyloxy-9-dehydro-7-deamino-7-hydroxy-porfiromycin.

12. A mitomycin of claim 1 represented by the general formula

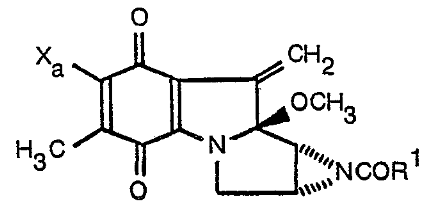

wherein Xa is amino or alkoxy having 1 to 4 carbon atoms and $R^1$ has the same meaning as defined above.

13. A process for production of a mitomycin selected from 10-decarbamoyloxy-9-dehydro-mitomycin B and 10-decarbamoyloxy-9-dehydro-porfiromycin which comprises culturing *Streptomyces caespitosus* ATCC 27422 in a nutrient medium and recovering at least one of said mitomycins accumulated in the culture liquor.

**Patentansprüche**

1. Ein Mitomycin, das durch die allgemeine Formel

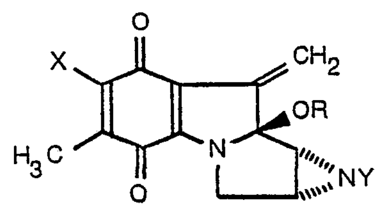

dargestellt ist,
worin X Amino, Hydroxy oder Alkoxy mit 1—4 Kohlenstoffatomen, Y Wasserstoff, Methyl, —$COR^1$ oder —$CH_2CH_2Z$,
$R^1$ Wasserstoff, Alkyl mit 1—3 Kohlenstoffatomen, Chloromethyl, Phenyl oder p-Methoxyphenyl,
Z Alkoxycarbonyl mit 2—5 Kohlenstoffatomen, —$COR^4$ oder Cyano,
$R^4$ Wasserstoff oder Alkyl mit 1—3 Kohlenstoffatomen,
R Wasserstoff oder Alkyl mit 1—4 Kohlenstoffatomen bedeuten.

2. Ein Mitomycin nach Anspruch 1, das durch die allgemeine Formel

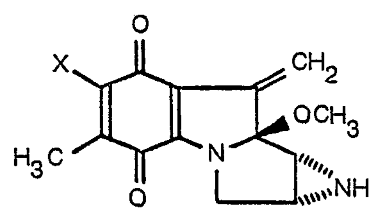

dargestellt ist,
in der X die gleiche Bedeutung hat, wie oben definiert.

3. Ein Mitomycin nach Anspruch 2, das durch die Formel

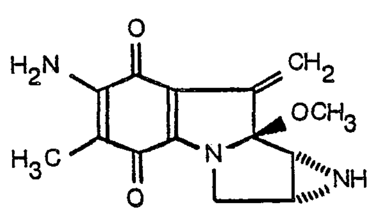

dargestellt ist,
d.h. 10-Decarbamoyloxy-9-Dehydro-Mitomycin C.

4. Ein Mitomycin nach Anspruch 2, das durch die Formel

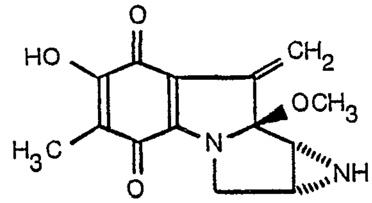

dargestellt ist,
d.h. 7-Deamino-10-Decarbamoyloxy-9-Dehydro-7-Hydroxymitomycin C.

5. Ein Mitomycin nach Anspruch 2, das durch die Formel

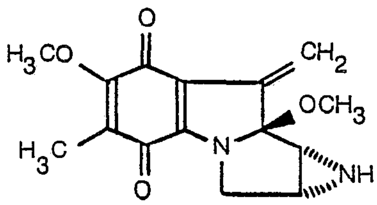

dargestellt ist,
d.h. 10-Decarbamoyloxy-9-Dehydro-Mitomycin A.

6. Ein Mitomycin nach Anspruch 1, das durch die allgemeine Formel

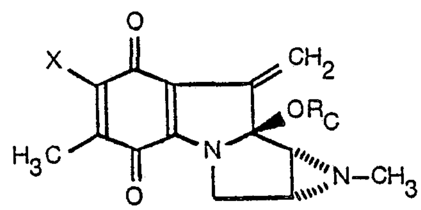

dargestellt ist,
in der X und $R_c$ die gleiche Bedeutung haben, wie oben definiert.

7. Ein Mitomycin nach Anspruch 6, das durch die Formel

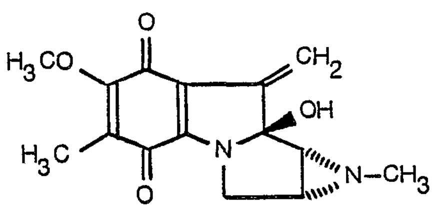

dargestellt ist,
d.h. 10-Decarbamoyloxy-9-Dehydro-Mitomycin B.

8. Ein Mitomycin nach Anspruch 6, das durch die Formel

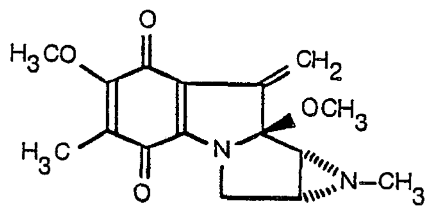

dargestellt ist,
d.h. 9a-O-Methyl-10-Decarbamoyloxy-9-Dehydro-Mitomycin B.

9. Ein Mitomycin nach Anspruch 6, das durch die Formel

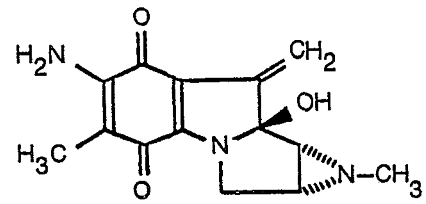

dargestellt ist,
d.h. 7-Amino-10-Decarbamoyloxy-9-Dehydro-7-Demethody-Mitomycin B.

10. Ein Mitomycin nach Anspruch 6, das durch die Formel

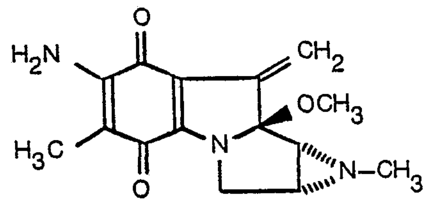

dargestellt ist,
d.h. 10-Decarbamoyloxy-9-Dehydro-Porfiromycin.

11. Ein Mitomycin nach Anspruch 6, das durch die Formel

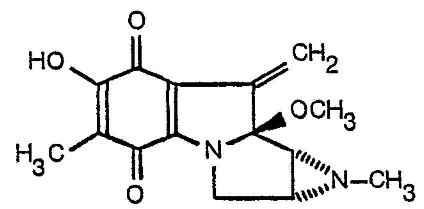

dargestellt ist,
d.h. 10-Decarbamoyloxy-9-Dehydro-7-Deamino-7-Hydroxy-Porfiromycin.

12. Ein Mitomycin nach Anspruch 1, das durch die allgemeine Formel

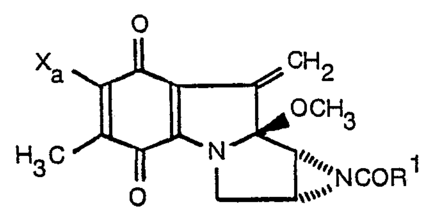

dargestellt ist, in der Xa ein Amino oder Alkoxy mit 1 bis 4 Kohlenstoffatomen und $R^1$ die gleiche Bedeutung hat, wie oben definiert.

13. Ein Verfahren zur Herstellung eines aus 10-Decarbamoyloxy-9-Dehydro-Mitomycin B und 10-Decarbamoyloxy-9-Dehydro-Porfiromycin ausgewählten Mitomycins, das die Züchtung *Streptomyces caespitosus* ATCC 27422 in einem Nährmedium und die Wiedergewinnung von mindestens einem der in dem flüssigen Nährboden angesammelten genannten Mitomycinarten umfasst.

## Revendications

1. Mitomycine représentée par la formule générale:

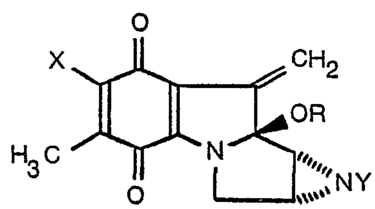

dans laquelle X désigne un groupement amino, hydroxy ou alcoxy ayant 1 à 4 atomes de carbone, Y désigne hydrogène, méthyle, —$COR^1$ ou —$CH_2CH_2Z$, dans lesquelles

$R_1$ désigne hydrogène, alkyle ayant de 1 à 3 atomes de carbone, chlorométhyle, phényle ou p-méthoxyphényle,

Z désigne alcoxycarbonyle ayant 2 à 5 atomes de carbone, —$COR^4$ ou cyano,

$R^4$ désignant hydrogène ou alkyle ayant 1 à 3 atomes de carbone,

R désigne hydrogène ou un groupement alkyle ayant 1 à 4 atomes de carbone.

2. Mitomycine selon la revendication 1, représentée par la formule générale:

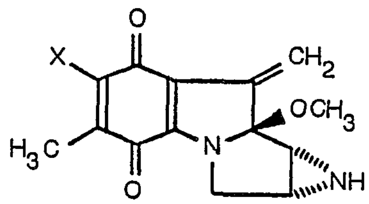

dans laquelle X a les mêmes significations que celles définies ci-dessus.

3. Mitomycine selon la revendication 2, représentée par la formule:

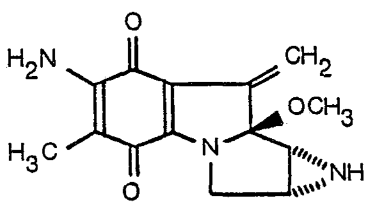

c'est-à-dire la 10-décarbamoyloxy-9-déhydro-mitomycine C.

4. Mitomycine selon la revendication 2, représentée par la formule générale:

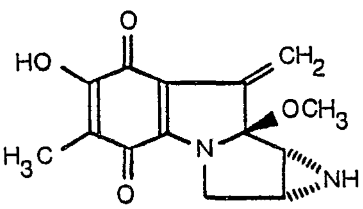

c'est-à-dire la 7-déamino-10-décarbamoyloxy-9-déhydro-7-hydroxymitomycine C.

5. Mitomycine selon la revendication 2, représentée par la formule:

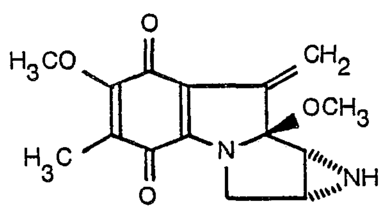

c'est-à-dire la 10-décarbamoyloxy-9-déhydro-mitomycine A.

6. Mitomycine selon la revendication 1, représentée par la formule générale:

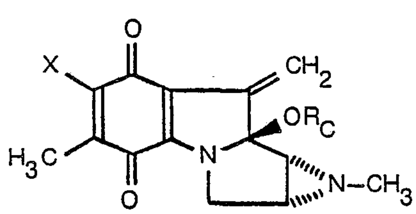

dans laquelle X et $R_C$ ont les mêmes significations que celles définies ci-dessus.

7. Mitomycine selon la revendication 6, représentée par la formule:

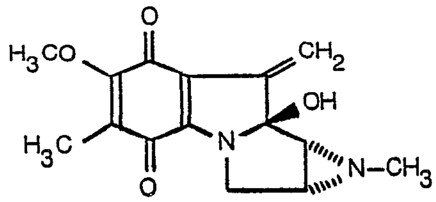

c'est-à-dire la 10-décarbamoyloxy-9-déhydro-mitomycine B.

8. Mitomycine selon la revendication 6, représentée par la formule:

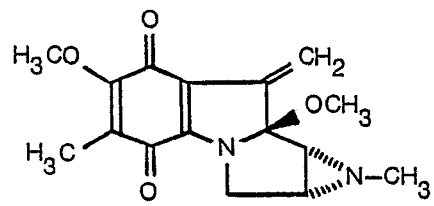

c'est-à-dire la 9a-O-méthyl-10-décarbamoyloxy-9-déhydro-mitomycine B.

9. Mitomycine selon la revendication 6, représentée par la formule:

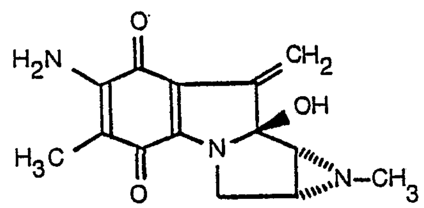

c'est-à-dire la 7-amino-10-décarbamoyloxy-9-déhydro-7-déméthoxymitomycine B.

10. Mitomycine selon la revendication 6, représentée par la formule:

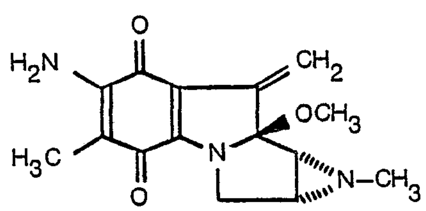

c'est-à-dire la 10-décarbamoyloxy-9-déhydro-porfiromycine.

11. Mitomycine selon la revendication 6, représentée par la formule:

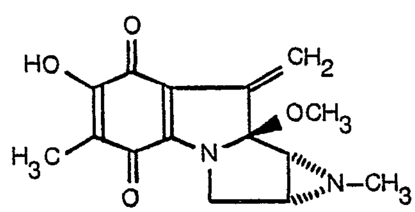

c'est-à-dire la 10-décarbamoyloxy-9-déhydro-7-déamino-7-hydroxy-porfiromycine.

12. Mitomycine selon la revendication 1, représentée par la formule générale:

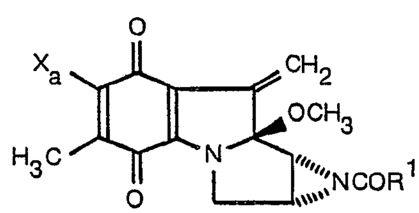

dans laquelle Xa désigne amino ou alcoxy ayant 1 à 4 atomes de carbone et $R^1$ a la même signification que celle définie ci-dessus.

13. Procédé de production d'une mitomycine choisie parmi la 10-décarbamoyloxy-9-déhydro-mitomycine B et la 10-décarbamoyloxy-9-déhydro-porfiromycine qui consiste à cultiver *Streptomyces caespitosus* ATCC 27422 dans un milieu nutritif et à récupérer au moins l'une desdites mitomycines accumulée dans la liqueur de culture.

FIG. 1

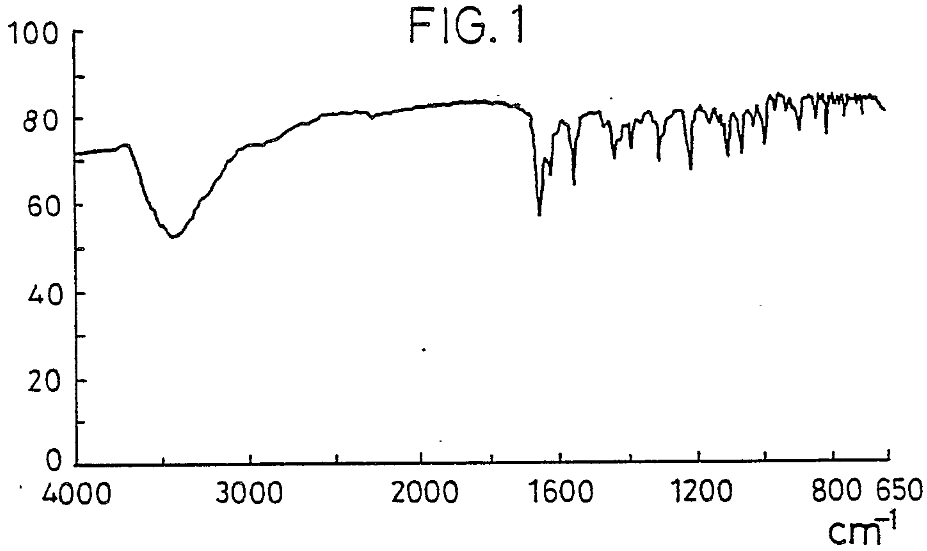
100
80
60
40
20
0
4000
3000
2000
1600
1200
800 650
cm⁻¹

FIG. 2

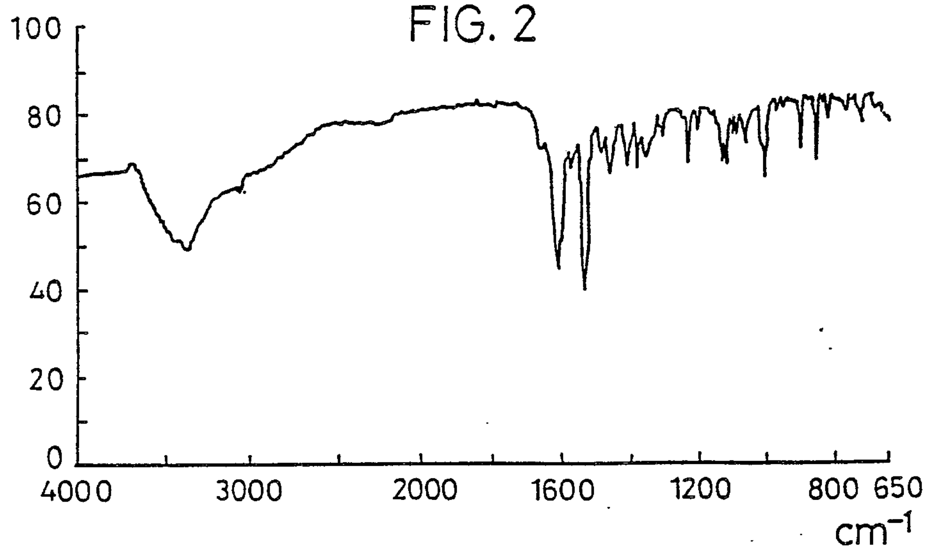
100
80
60
40
20
0
4000
3000
2000
1600
1200
800 650
cm⁻¹

FIG. 3

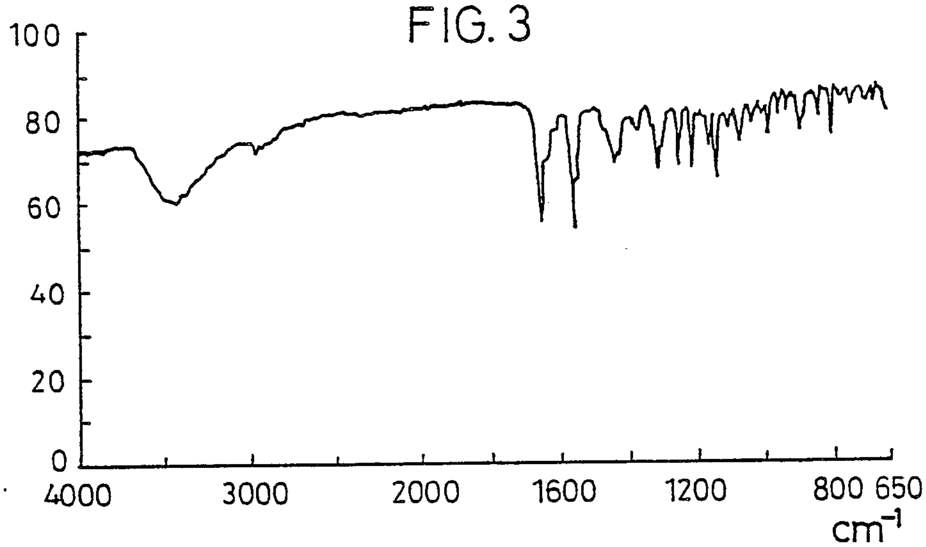
100
80
60
40
20
0
4000
3000
2000
1600
1200
800 650
cm⁻¹

FIG. 4

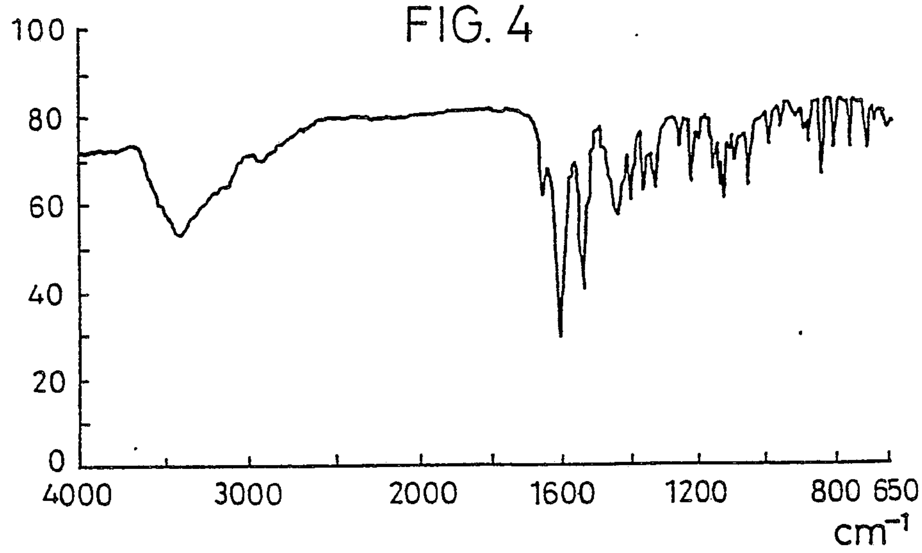
100
80
60
40
20
0
4000
3000
2000
1600
1200
800 650
cm⁻¹

FIG. 5

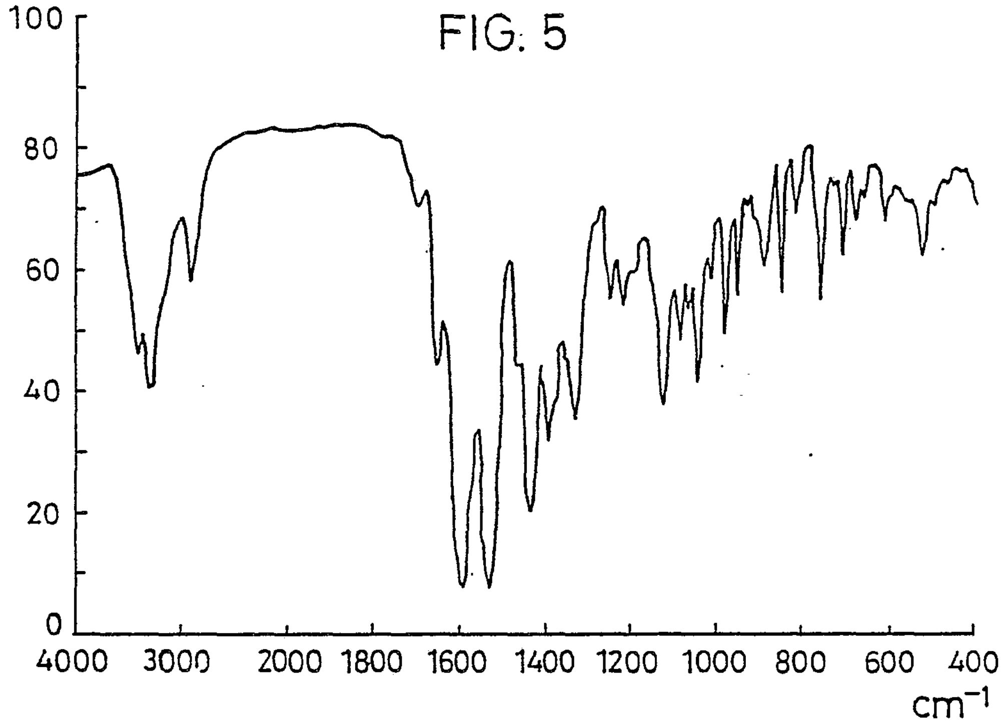
100
80
60
40
20
0
4000
3000
2000
1800
1600
1400
1200
1000
800
600
400
cm⁻¹

FIG. 6

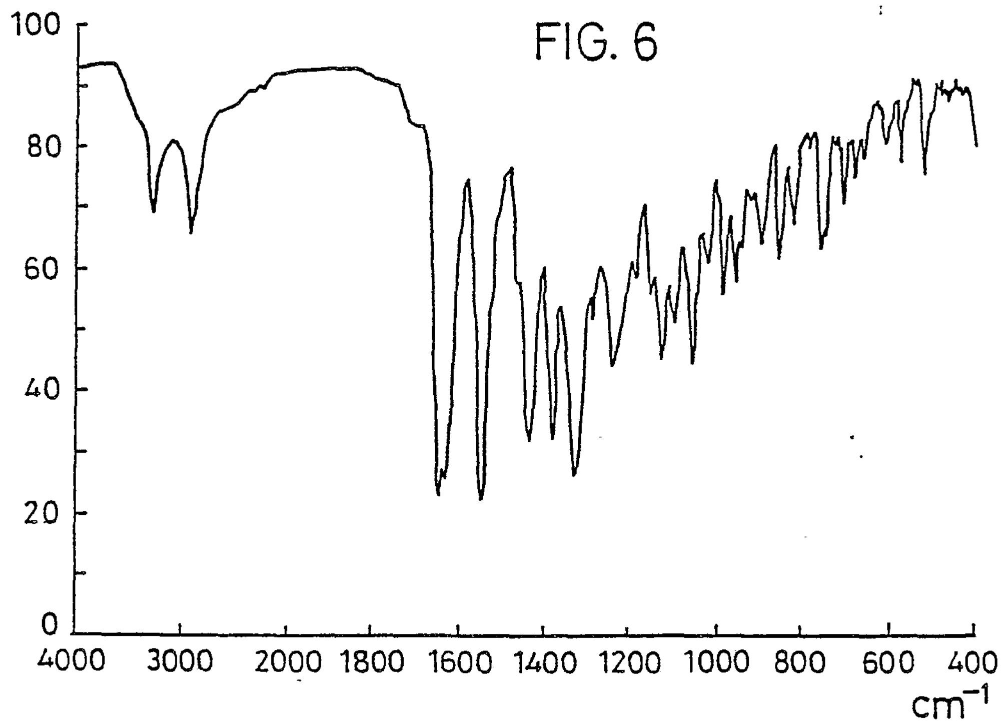
100
80
60
40
20
0
4000
3000
2000
1800
1600
1400
1200
1000
800
600
400
cm⁻¹

FIG. 7

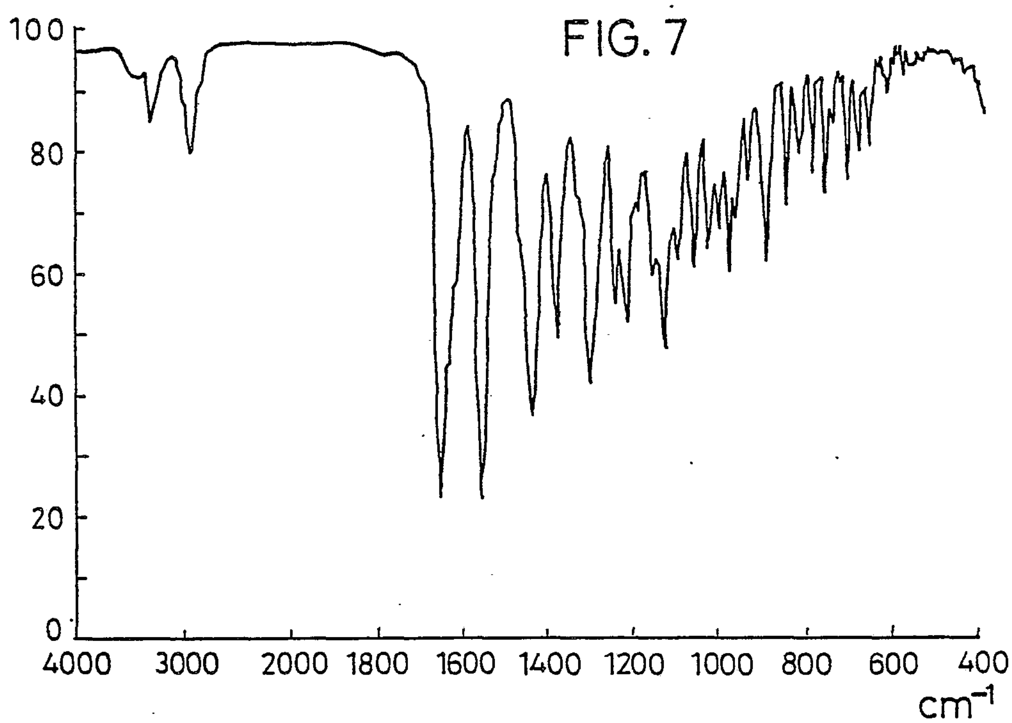
100
80
60
40
20
0
4000
3000
2000
1800
1600
1400
1200
1000
800
600
400
cm⁻¹

FIG. 8

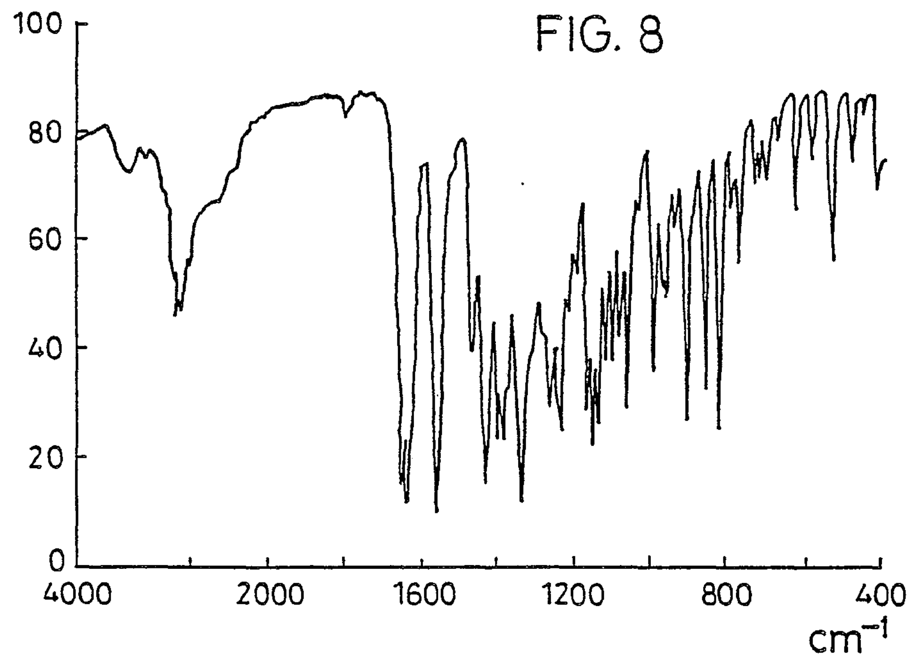
100
80
60
40
20
0
4000
2000
1600
1200
800
400
cm⁻¹